# EUROPEAN PATENT APPLICATION

(11) **EP 4 130 843 A1**
(43) Date of publication of application: **08.02.2023**
(21) Application number: 21779707.5
(22) Date of filing: 10.03.2021
(51) Int. Cl.: G02B 21/36, C12M 1/34, G02B 21/18

(54) **MICROSCOPE SYSTEM, PROJECTION UNIT, AND SPERM SORTING ASSISTANCE METHOD**

(30) Priority: 31.03.2020 JP 2020063284
(71) Applicant: Evident Corporation, Nagano 399-0495 (JP)
(72) Inventor: KIMURA, Madoka, Kamiina-gun, Nagano 399-0495 (JP); YAMANE, Takuto, Kamiina-gun, Nagano 399-0495 (JP); HATTORI, Toshiyuki, Kamiina-gun, Nagano 399-0495 (JP)
(74) Representative: Gunzelmann, Rainer
(86) International application number: PCT/JP2021/009685
(87) International publication number: WO 2021/200003

(57) **Abstract**

A microscope system 1 includes a microscope 100, an photographing device 143 that acquires a photographed image of a sample containing sperm placed on a stage 111 of the microscope 100, an image processing device 200 that generates an auxiliary image including auxiliary information contributing to sperm sorting on the basis of the photographed image acquired by the photographing device 143, and a projection device 153 that is a superimposition device that superimposes the auxiliary image generated by the image processing device 200 on an optical image of the sample formed on an image surface on an optical path of the microscope 100.

## Description

### Technical Field

The present disclosure relates to a microscope system, a projection unit, and a sperm sorting support method.

### Background Art

At present, with the progress of late marriage and late childbirth, the number of patients who receive infertility treatment is increasing year by year, and a demand for assisted reproductive technology (ART) is also increasing.

The ART is a generic term for a technique for fertilizing an egg and a sperm extracted from a human in vitro, such as in vitro fertilization (IVF) and microinsemination, and is distinguished from general artificial insemination in which the collected sperm is injected into a uterus and fertilized with the egg in the body.

A technique related to the ART is described in, for example, Patent Literature 1. Patent Literature 1 describes a microscope suitable for intracytoplasmic sperm injection (ICSI) used in microinsemination, which is a type of ART. The ICSI is a method of directly injecting sperm into an egg by piercing an injection pipette containing sperm into the egg fixed with a holding pipette.

### Citation List

### Patent Literature

Patent Literature 1: WO 2012/150689 A

### Summary of Invention

### Technical Problem

Incidentally, in order to increase a success rate of ICSI, it is important to sort sperm and inject sperm suitable for fertilization into an egg. However, whether or not the sperm obtained by the sorting operation is of high quality is largely based on the experience of an embryo cultivator who is an operator, and a difference in fertilization rate is likely to occur between embryo cultivators.

In view of the above circumstances, an object according to one aspect of the present invention is to provide a technique for easily grasping high-quality sperm in sperm sorting operation.

### Solution to Problem

According to an aspect of the present invention, there is provided a microscope system including: a microscope; a photographing device that acquires a photographed image of a sample containing sperm placed on a stage of the microscope; an image processing device that generates an auxiliary image including auxiliary information contributing to sperm sorting on the basis of the photographed image acquired by the photographing device; and a superimposition device that superimposes the auxiliary image generated by the image processing device on an optical image of the sample formed on an image surface on an optical path of the microscope.

According to an aspect of the present invention, there is provided a projection unit used by being mounted on a microscope, including: an image processing device that generates an auxiliary image including auxiliary information contributing to sperm sorting on the basis of a photographed image of a sample including sperm placed on a stage of the microscope; and a superimposition device that superimposes the auxiliary image generated by the image processing device on an optical image of the sample formed on an image surface on an optical path of the microscope.

According to an aspect of the present invention, there is provided a sperm sorting support method including: acquiring a photographed image of a sample including sperm placed on a stage of a microscope; generating an auxiliary image including auxiliary information contributing to sperm sorting on the basis of the acquired photographed image; and superimposing the generated auxiliary image on an optical image of the sample formed on an image surface on an optical path of the microscope.

### Advantageous Effects of Invention

According to the above aspect, it is possible to easily grasp high-quality sperm in a sperm sorting operation.

### Brief Description of Drawings

FIG. 1 is a diagram illustrating a configuration of a microscope system 1.
FIG. 2 is a diagram illustrating a configuration of a microscope 100.
FIG. 3 is a diagram illustrating a configuration of an operation unit of an input device 50.
FIG. 4 is a diagram illustrating a configuration of an image processing device 200.
FIG. 5 is a flowchart illustrating an example of a procedure of creating a learning data set.
FIG. 6 is a flowchart illustrating an example of learning processing.
FIG. 7 is a flowchart illustrating another example of a procedure of creating the learning data set.
FIG. 8 is a diagram showing an example of a screen for creating the learning data set.
FIG. 9 is a flowchart illustrating an example of sperm sorting support processing.
FIG. 10 is a flowchart illustrating an example of auxiliary image generation processing.
FIG. 11 is a diagram for explaining image processing on a photographed image.
FIG. 12 is a diagram for explaining an image projected on an image surface.
FIG. 13 is a flowchart illustrating an example of a procedure of ICSI.
FIG. 14 is a diagram illustrating a configuration of a drop formed as a sample 300 in a Petri dish 310.
FIG. 15 is a flowchart illustrating an example of a sperm sorting procedure.
FIG. 16 is a flowchart illustrating another example of the auxiliary image generation processing.
FIG. 17 is a diagram for describing a configuration example of an auxiliary image setting window.
FIG. 18 is a diagram illustrating an example of a relationship between auxiliary image setting and an image viewed from an eyepiece 101.
FIG. 19 is a diagram illustrating another example of the relationship between the auxiliary image setting and the image viewed from the eyepiece 101.
FIG. 20 is a diagram illustrating still another example of the relationship between the auxiliary image setting and the image viewed from the eyepiece 101.
FIG. 21 is a diagram illustrating still another example of the relationship between the auxiliary image setting and the image viewed from the eyepiece 101.
FIG. 22 is a diagram illustrating still another example of the relationship between the auxiliary image setting and the image viewed from the eyepiece 101.
FIG. 23 is a diagram illustrating still another example of the relationship between the auxiliary image setting and the image viewed from the eyepiece 101.
FIG. 24 is a diagram illustrating still another example of the relationship between the auxiliary image setting and the image viewed from the eyepiece 101.
FIG. 25 is a diagram illustrating a configuration of a microscope 400.
FIG. 26 is a diagram illustrating a configuration of a microscope system 2.
FIG. 27 is a diagram illustrating still another example of the relationship between the auxiliary image setting and the image viewed from the eyepiece 101.
FIG. 28 is a diagram illustrating still another example of the relationship between the auxiliary image setting and the image viewed from the eyepiece 101.
FIG. 29 is a diagram for describing an advantage of bar display.
FIG. 30 is another diagram for describing the advantage of the bar display.
FIG. 31 is a diagram for explaining a desirable modification of the display format of the auxiliary image.

### Description of Embodiments

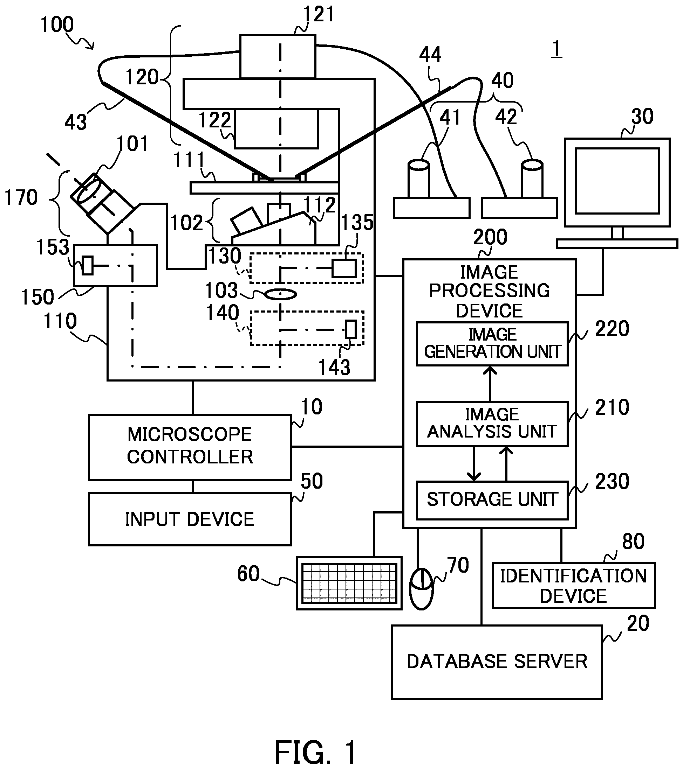

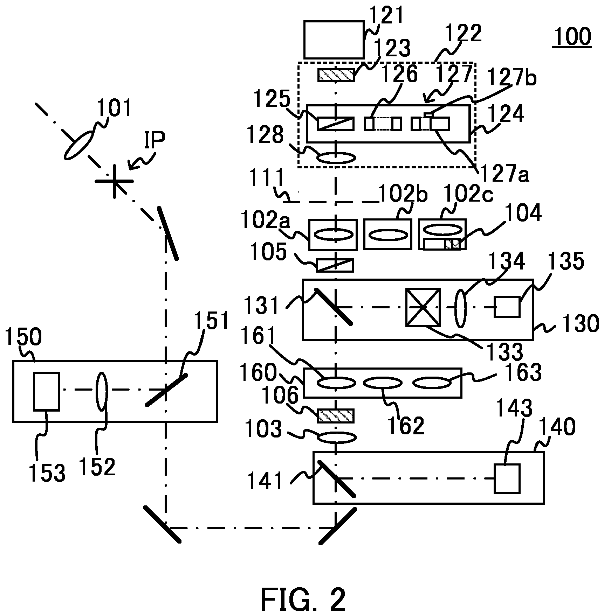

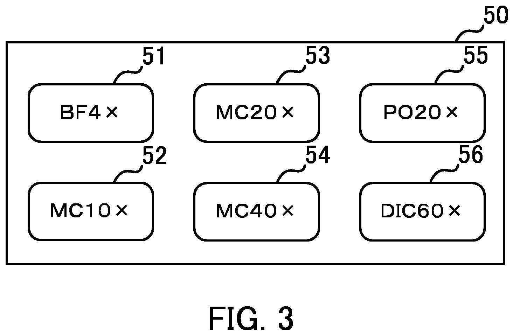

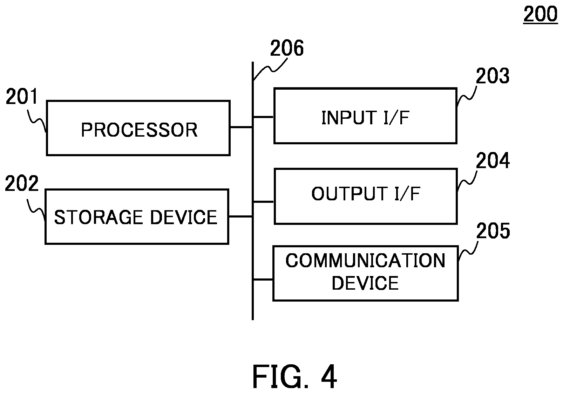

FIG. 1 is a diagram illustrating a configuration of a microscope system 1. FIG. 2 is a diagram illustrating a configuration of the microscope 100. FIG. 3 is a diagram illustrating a configuration of an operation unit of input device 50. FIG. 4 is a diagram illustrating a configuration of an image processing device 200.

The microscope system 1 is a system for observing a sample while looking into an eyepiece 101. Specifically, the microscope system 1 is an inverted microscope system provided with a transmitted illumination system 120 used for microinsemination, particularly sperm sorting, and is used by, for example, an embryo cultivator. The sample to be observed by the microscope system 1 is a sperm suspension containing sperm accommodated in a Petri dish or the like at the time of sperm sorting operation.

The microscope system 1 includes at least a microscope 100, a photographing device 143, a projection device 153 as an example of a superimposition device, and an image processing device 200. The microscope system 1 projects an auxiliary image on an image surface on which an optical image of a sample is formed by the optical system of the microscope 100 using the projection device 153, thereby superimposing the auxiliary image on the optical image. The auxiliary image includes information (hereinafter, it is referred to as auxiliary information.) contributing to sperm sorting. The auxiliary image is created by the image processing device 200 based on the photographed image of the sample captured by a photographing device 143.

As a result, the embryo cultivator of the microscope system 1 observing the sample while looking into the eyepiece 101 can obtain information contributing to sperm sorting without taking his/her eyes off the eyepiece 101, so that it is possible to accurately specify and collect good sperm in a short time. Therefore, according to the microscope system 1, it is possible to support the sperm sorting operation of the embryo cultivator.

Hereinafter, a specific example of the configuration of the microscope system 1 will be described in detail with reference to FIGS. 1 to 4. As illustrated in FIG. 1, the microscope system 1 includes the microscope 100 including the photographing device 143 and the projection device 153, and the image processing device 200. Further, the microscope system 1 includes a microscope controller 10, a display device 30, a plurality of input devices (Input device 40, input device 50, input device 60, input device 70), and an identification device 80. In addition, the microscope system 1 is connected to a database server 20 in which various data are stored.

The microscope 100 is an inverted microscope including the eyepiece 101, and includes a microscope body 110, a plurality of objectives 102, a stage 111, a transmitted illumination system 120, and an ocular lens barrel 170 attached to the microscope body 110 as illustrated in FIG. 1. In addition, as described later, the microscope 100 includes a modulation element for visualizing an unstained sample such as sperm or egg in each of an illumination optical path and an observation optical path. A user such as an embryo cultivator can observe a sample by four microscopy methods of bright field (BF) observation, polarized light (PO) observation, differential interference contrast (DIC) observation, and modulated contrast (MC) observation using the microscope 100. The modulated contrast observation is also referred to as relief contrast (RC) observation.

The plurality of objectives 102 are attached to a nosepiece 112. As illustrated in FIG. 2, the plurality of objectives 102 includes an objective 102a for BF observation, an objective 102b for PO observation and DIC observation, and an objective 102c for MC observation. The objective 102c includes a modulator 104. The modulator 104 includes three regions (for example, a region having a transmittance of about 100%, a region having a transmittance of about 5%, and a region having a transmittance of about 0%) having different transmittances.

Although FIG. 2 illustrates three objectives according to the microscopy method, the plurality of objectives 102 may include a plurality of objectives having different magnifications for each microscopy method. Hereinafter, a case where a 4 times objective for BF observation, 10 times, 20 times, and 40 times objectives for MC observation, a 20 times objective for PO observation, and a 60 times objective for DIC observation are included will be described as an example.

The nosepiece 112 is a switching device that switches the objective arranged on the optical path among the plurality of objectives 102. The nosepiece 112 switches the objective arranged on the optical path according to the microscopy method and the observation magnification. The objective disposed on the optical path by the nosepiece 112 guides the transmitted light transmitted through the sample to the eyepiece 101.

The sample put in a container is placed on the stage 111. The container is, for example, a Petri dish, and the sample includes germ cells such as sperm and egg. The stage 111 moves in an optical axis direction of the objective 102 arranged on the optical path and in a direction orthogonal to the optical axis of the objective 102. The stage 111 may be a manual stage or an electric stage.

The transmitted illumination system 120 illuminates the sample placed on the stage 111 from above the stage 111. As illustrated in FIGS. 1 and 2, the transmitted illumination system 120 includes a light source 121 and a universal condenser 122. The light source 121 may be, for example, a light emitting diode (LED) light source or a lamp light source such as a halogen lamp light source.

As illustrated in FIG. 2, the universal condenser 122 includes a polarizer 123 (first polarizing plate), a plurality of optical elements housed in a turret 124, and a condenser lens 128. The polarizer 123 is used in MC, PO and DIC observations. The turret 124 accommodates a plurality of optical elements to be switched and used according to the microscopy method. The DIC prism 125 is used in DIC observation. An aperture plate 126 is used for BF observation and PO observation. The optical element 127 is a combination of a slit plate 127a which is a light shielding plate in which a slit is formed and a polarizing plate 127b (second polarizing plate) arranged to cover a part of the slit, and is used in MC observation.

The ocular lens barrel 170 includes the eyepiece 101. A tube lens 103 is disposed between the eyepiece 101 and the objective 102. The tube lens 103 forms an optical image of the sample on an image surface IP between the eyepiece 101 and the tube lens 103 on the basis of the transmitted light. An auxiliary image to be described later is also formed on the image surface IP on the basis of the light from the projection device 153. As a result, the auxiliary image is superimposed on the optical image on the image surface IP. The user of the microscope system 1 uses the eyepiece 101 to observe a virtual image of an image in which an auxiliary image is superimposed on an optical image formed on the image surface IP.

As illustrated in FIG. 1, the microscope body 110 includes a laser assisted hatching unit 130, a photographing unit 140, and a projection unit 150. As illustrated in FIG. 2, the microscope body 110 includes an intermediate magnification unit 160. Further, the microscope body 110 includes a DIC prism 105 and an analyzer 106 so as to be insertable into and removable from the optical path.

As illustrated in FIG. 2, the laser assisted hatching unit 130 is a laser unit disposed between the objective 102 and the tube lens 103. The laser assisted hatching unit 130 irradiates the sample with the laser beam by introducing the laser beam from between the objective 102 and the tube lens 103. More specifically, for example, the laser assisted hatching unit 130 irradiates a transparent zone surrounding an embryo grown from the fertilized egg with laser light. The laser assisted hatching unit 130 includes a splitter 131, a scanner 133, a lens 134, and a laser 135. The splitter 131 is, for example, a dichroic mirror. The scanner 133 is, for example, a galvano scanner, and adjusts the irradiation position of the laser light in a direction orthogonal to the optical axis of the objective 102. The lens 134 converts the laser beam into a parallel light flux. As a result, the laser light is condensed on the sample by the objective 102.

As illustrated in FIG. 2, the photographing unit 140 includes a splitter 141 and the photographing device 143 that acquires a photographed image of a sample based on transmitted light. The photographing unit 140 is disposed between the tube lens 103 and the eyepiece 101. The splitter 141 is, for example, a half mirror. The tube lens 103 forms an optical image of a sample on a light receiving surface of an imaging element included in the photographing device 143. The photographing device 143 is, for example, a digital camera, and the imaging element included in the photographing device 143 is, for example, a charge coupled device (CCD) image sensor, a complementary metal-oxide-semiconductor (CMOS) image sensor, or the like. The imaging element detects light from the sample and converts the detected light into an electric signal by photoelectric conversion. The photographing unit 140 outputs the photographed image acquired by the photographing device 143 to the image processing device 200.

Note that the microscope system 1 is used for sperm sorting, but the details of sperm, for example, the portion of the tail is about Φ0.5 um. In order to identify this on the image, a pixel pitch of Φ0.5 µm or less when projected onto the object surface is required. That is, a pitch of a pixel projection image on the object surface calculated by dividing the pixel pitch by a total magnification (= magnification of objective 102 × magnification of intermediate magnification unit 160 × magnification of camera adapter (not illustrated)) is required to be Φ0.5 µm or less. For example, in the case of a combination of an objective of 20 times, an intermediate magnification lens of 2 times, and a camera adapter of 0.25 times, the total magnification is 10 times. In this case, by using the digital camera for a microscope having a pixel pitch of 3.45 µm, the pitch of the pixel projection image on the object surface becomes 0.345 µm, and a tail portion of sperm can also be determined. Note that, in the actual selection of the digital camera, it is further noted that the region including the effective pixels has a size that satisfies the entire visual field.

The projection unit 150 is disposed between the tube lens 103 and the eyepiece 101. As illustrated in FIG. 2, projection unit 150 includes a splitter 151, a lens 152, and a projection device 153. The splitter 151 is, for example, a half mirror. The projection device 153 projects the auxiliary image generated by the image processing device 200. More specifically, the lens 152 condenses the light from the projection device 153 on the image surface of the tube lens 103, that is, at the same position as the image surface IP on which the optical image is formed, so that the projection device 153 projects the auxiliary image on the optical image formed on the image surface IP on the optical path.

For example, the size from a head to a tail of the sperm is about 60 um, and the short side of the size of the head is about 3 um. When this is projected on the image surface IP closer to the object side than the eyepiece 101 by a combination of an objective of 20 times for MC observation and an intermediate magnification lens of 1 time, the image of sperm has a size of 1.2 mm × 0.06 mm. For example, as will be described later, when an auxiliary image including a bounding box surrounding a sperm image is created to indicate the position of sperm, the auxiliary image becomes a rectangle of about 1.5 mm × 0.1 mm at minimum. In order to project the gap of 0.1 mm at the minimum so that the gap can be recognized in the field of view of the eyepiece, when a projection magnification of lens 152 is 1 time, projection device 153 including light emitting elements (in the case of a single color) having a pitch of 0.05 mm or less may be used. As a result, it is possible to display the auxiliary image with which the user can recognize the gap of 0.1 mm.

Further, the projection device 153 not only satisfies the visual field of eyepiece 101 having visual field number Φ22 but also projects the auxiliary image in a range larger than the visual field number Φ23. Specifically, in a case where the projection magnification of the lens 152 is 1 time, the projection device 153 having an effective light emission area corresponding to a range of the number of visual fields Φ23 or more is used. As a result, the sperm in the peripheral portion of the visual field that has entered the visual field from outside the visual field of the eyepiece 101 is also included in the auxiliary image. Therefore, it is possible to fully recognize good sperm from all the sperm in the visual field including the peripheral portion of the visual field of the eyepiece 101. Note that, in this case, the effective pixel region of the photographing device 143 also needs to correspond to a range of the number of visual fields Φ23 or more in the eyepiece.

The intermediate magnification unit 160 is disposed between the objective 102 and the tube lens 103. As illustrated in FIG. 2, the intermediate magnification unit 160 includes a plurality of lenses (lens 161, lens 162, lens 163), and changes the magnification of the optical image formed on the image surface by switching the lenses arranged on the optical path between the lenses. By using the intermediate magnification unit 160, it is possible to change the magnification of the optical image without switching the objective 102 located near the sample.

The DIC prism 105 and the analyzer 106 are disposed between the objective 102 and the tube lens 103. The DIC prism 105 is used in DIC observation. The analyzer 106 is used in PO observation and DIC observation.

In the microscope 100, when MC observation is performed, the polarizer 123 and the optical element 127 are arranged on an illumination optical path as modulation elements (hereinafter, referred to as a first modulation element.) that modulate illumination light applied to a sample, and a modulator 104 is arranged on an observation optical path as modulation elements (hereinafter, referred to as a second modulation element.) that modulate transmitted light. Further, when the PO observation is performed, the polarizer 123 is arranged on the illumination optical path as the first modulation element, and the analyzer 106 is arranged on the observation optical path as the second modulation element. When the DIC observation is performed, the polarizer 123 and the DIC prism 125 are arranged on the illumination optical path as the first modulation element, and the analyzer 106 and the DIC prism 105 are arranged on the observation optical path as the second modulation element. As a result, it is possible to visualize an unstained sample, and for example, it is possible to sort sperm.

The microscope controller 10 is a device that controls the microscope 100. The microscope controller 10 is connected to the image processing device 200, the input device 50, and the microscope 100, and controls the microscope 100 in accordance with a command from the image processing device 200 or the input device 50.

The display device 30 is, for example, a display device such as a liquid crystal display, a plasma display, an organic EL display, a CRT display, or an LED matrix panel.

The input device 40 includes a handle 41 and a handle 42. Operation of a micromanipulator (not illustrated) that moves the pipette 43 and a pipette 44 is controlled by operating the handle 41 and the handle 42. The pipette 43 and the pipette 44 are used to manipulate samples in microinsemination operations, including sperm sorting. The pipette 43 is, for example, a holding pipette, and the pipette 44 is, for example, an injection pipette.

The input device 50 is a hand switch device for changing settings related to the microscopy method and the observation magnification of the microscope 100. As illustrated in FIG. 3, the input device 50 includes, for example, six buttons (buttons 51 to 56), and the user can quickly switch the setting of the microscope 100 only by pressing these buttons.

When the user presses the button 51, the setting of the microscope 100 is switched to the setting of BF observation (hereinafter, it is referred to as BF 4 × observation.) at an observation magnification of 4 times. When the user presses the button 52, the setting of the microscope 100 is switched to the setting of MC observation (hereinafter, it is referred to as MC 10 × observation.) at an observation magnification of 10 times. When the user presses the button 53, the setting of the microscope 100 is switched to the setting of MC observation (hereinafter, it is referred to as MC 20 × observation.) at an observation magnification of 20 times. When the user presses the button 54, the setting of the microscope 100 is switched to the setting of MC observation (hereinafter, it is referred to as MC 40 × observation.) at an observation magnification of 40 times. When the user presses the button 55, the setting of the microscope 100 is switched to the setting of PO observation (hereinafter, it is referred to as PO 20 × observation.) at an observation magnification of 20 times. When the user presses the button 56, the setting of the microscope 100 is switched to the setting of DIC observation (hereinafter, it is referred to as DIC 60 × observation.) at an observation magnification of 60 times.

The input device 60 is a keyboard. The input device 70 is a mouse. The input device 60 and the input device 70 are connected to the image processing device 200. Note that the microscope system 1 may include other input devices (not illustrated) such as a touch panel, a sound input device, and a foot pedal.

The identification device 80 is a device that acquires identification information added to a sample. Note that adding to the sample includes, for example, a case where the identification information is attached to a container that stores the sample. The identification information is information for identifying the sample, and more specifically, for example, is information for specifying a patient who has provided the sample. The identification device 80 may be, for example, a barcode reader, an RFID (registered trademark) reader, a QR code (registered trademark) reader, or the like.

The image processing device 200 generates the auxiliary image based on the photographed image acquired by the photographing device 143. The generated auxiliary image is output to the projection device 153 of the microscope 100 directly or via the microscope controller 10. As illustrated in FIG. 1, the image processing device 200 is connected to the microscope 100, the microscope controller 10, the display device 30, the input device 60, the input device 70, and the identification device 80. The image processing device 200 is also connected to the database server 20.

The image processing device 200 includes an image analysis unit 210, an image generation unit 220, and a storage unit 230 as functional components related to generation of an auxiliary image including auxiliary information contributing to sperm sorting.

The image analysis unit 210 performs image analysis including object detection for sperm for the photographed image and recommendation degree estimation for estimating a recommendation degree of sperm detected by the object detection. Specifically, the image analysis unit 210 performs, for example, object detection and recommendation degree estimation using a learned model stored in the storage unit 230. The object detection and the recommendation degree estimation may be performed using two or more learned models, or may be performed using a single learned algorithm. Note that the algorithm of the learned model is not particularly limited, but for example, a deep learning model such as SSD, YOLO, or FasterR-CNN may be used.

The image generation unit 220 generates an auxiliary image including auxiliary information contributing to sperm sorting on the basis of a result of image analysis by the image analysis unit 210. The auxiliary information includes information (hereinafter, it is referred to as recommendation degree information.) on the recommendation degree of the sperm generated on the basis of the result of the recommendation degree estimation. That is, the image generation unit 220 generates the auxiliary image including at least the recommendation degree information. Note that the recommendation degree information may be numerical information such as a DFI to be described later, or may be symbol information indicating a grade such as a DFI rank and a MM rank to be described later. The recommendation degree information may be a combination of one or more pieces of numerical information and one or more pieces of symbol information. The recommendation degree information may include at least one of at least one or more pieces of numerical information and one or more pieces of symbol information. At least a part of the recommendation degree information may be calculated on the basis of a history of results of recommendation degree estimation within a predetermined period. The auxiliary information may further include information (hereinafter, it is referred to as position information.) on the position of the sperm generated based on the result of the object detection. That is, image generation unit 220 may generate the auxiliary image including the position information in addition to the recommendation degree information.

The recommendation degree estimation performed by the image analysis unit 210 may include integrity estimation for estimating DNA integrity of sperm detected by object detection. In that case, the recommendation degree information may include integrity information on DNA integrity of the sperm, the integrity information being generated based on a result of the integrity estimation. Note that the integrity information desirably includes at least one of a DFI to be described later and a rank according to the DFI. Further, the DFI or DFI rank desirably includes at least two of latest information, average information, and maximum information. Furthermore, the recommendation degree estimation performed by the image analysis unit 210 may include quality estimation for estimating the quality of at least one of the morphology and the motility of sperm detected by object detection. In this case, the recommendation degree information may include quality information on at least one of the morphological quality of sperm and the motility quality of sperm, which is generated on the basis of the result of the quality estimation.

In the conventional sperm sorting, the embryo cultivator determines the morphological quality of the sperm and the motile quality of the sperm based on the optical image seen from the eyepiece, and sorts good sperm mainly based on these qualities. This is because it is recognized that DNA integrity is an effective prognostic marker for evaluating sperm fertility, but it is difficult for humans to visually observe sperm to determine DNA integrity. On the other hand, for example, as described in Non-Patent Document 1 (Wang, Y. et al., Prediction of DNA Integrity from Morphological Parameters Using a Single-Sperm DNA Fragmentation Index Assay, ADVANCED SCIENCE, (2019)), there is a correlation between DNA integrity and sperm morphology. Thus, it is possible to estimate sperm integrity from an image of sperm. The integrity estimation performed by the image analysis unit 210 is performed using a learned model that has learned the DNA integrity of sperm with respect to the morphology of sperm extracted from the image using this fact.

The recommendation degree information included in the auxiliary image may have a mode according to the recommendation degree estimated by the recommendation degree estimation. More specifically, for example, the color may have a color corresponding to the recommendation degree estimated by the recommendation degree estimation. Similarly to the recommendation degree information, the position information included in the auxiliary image may have a mode according to the recommendation degree estimated by the recommendation degree estimation, and for example, may have a color according to the recommendation degree estimated by the recommendation degree estimation.

The storage unit 230 stores a learned model used in image analysis performed by the image generation unit 220. The storage unit 230 stores a learned model for object detection for sperm, and the image generation unit 220 performs object detection using the learned model stored in the storage unit 230. Furthermore, for example, in a case where the integrity information is generated by the image generation unit 220, a learned model in which the DNA integrity of sperm with respect to the morphology of sperm is learned is stored in the storage unit 230. The image generation unit 220 performs integrity estimation using the learned model stored in the storage unit 230. Furthermore, for example, in a case where the quality information is generated by the image generation unit 220, the storage unit 230 stores a learned model in which the sperm quality with respect to at least one of the sperm morphology and the sperm motility has been learned. The image generation unit 220 performs the quality estimation using the learned model stored in the storage unit 230. Note that the object detection, the integrity estimation, and the quality estimation may be performed using a single learned model stored in the storage unit 230, or may be performed using two or more learned models stored in the storage unit 230.

The image processing device 200 may be a general-purpose computer or a dedicated computer. The image processing device 200 is not particularly limited to this configuration, but may have, for example, a physical configuration as illustrated in FIG. 4. Specifically, the image processing device 200 may include a processor 201, a storage device 202, an input interface (I/F) 203, an output interface (I/F) 204, and a communication device 205, which may be connected to each other by a bus 206.

The processor 201 may include hardware, which may include, for example, at least one of a circuit for processing digital signals and a circuit for processing analog signals. The processor 201 may include, for example, one or more circuit devices (for example, IC) or one or more circuit elements (for example, a resistor and a capacitor) on a circuit board. The processor 201 may be a central processing unit (CPU). Furthermore, various types of processors including a graphics processing unit (GPU) and a digital signal processor (DSP) may be used as the processor 201. The processor 201 may be a hardware circuit including an application specific integrated circuit (ASIC) or a field-programmable gate array (FPGA). The processor 201 may include an amplifier circuit, a filter circuit, and the like for processing an analog signal. The processor 201 functions as the image analysis unit 210 and the image generation unit 220 described above by executing a program stored in the storage device 202.

The storage device 202 may include memory and/or other storage. The memory may be, for example, a random access memory (RAM). The memory may be a semiconductor memory such as a static random access memory (SRAM) or a dynamic random access memory (DRAM). The storage device 202 may be, for example, a register, a magnetic storage device such as a hard disk device, an optical storage device such as an optical disk device, an internal or external hard disk drive, a solid-state storage device, a CD-ROM, a DVD, other optical or magnetic disk storage devices, or other storage devices. The storage device 202 stores a program executed by the processor 201, a learned model, and other data, and functions as the storage unit 230 described above. Note that the storage device 202 is an example of a non-transitory computer-readable storage medium.

The input I/F 203 is connected to an input device operated by a user (for example, an embryo cultivator) of the microscope system 1, receives an operation signal corresponding to an operation on the input device, and outputs the operation signal to the processor 201.

The output I/F 204 is connected to the display device 30. The output I/F 204 may be further connected to a sound output device such as a speaker that outputs sound, a light emitting device such as a lamp or a light that outputs light, a vibration device such as a vibrator that outputs vibration, and the like (not illustrated).

The communication device 205 is a device that exchanges data with the microscope 100 and other devices. The communication device 205 may be a communication device that exchanges data in a wired manner or a communication device that exchanges data in a wireless manner. The program and the learned model stored in the storage device 202 may be acquired by the communication device 205 from another device via the Internet.

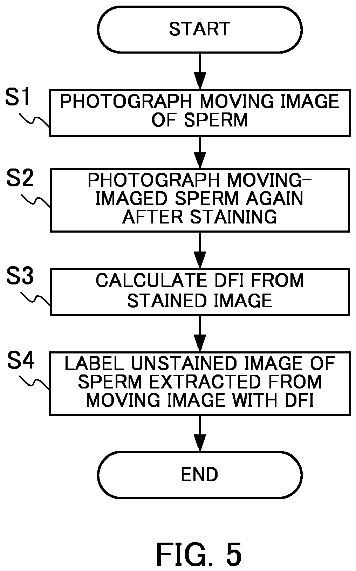

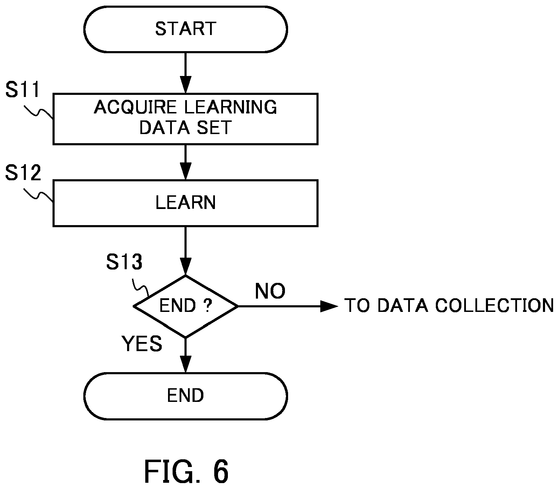

FIG. 5 is a flowchart illustrating an example of a procedure of creating a learning data set. FIG. 6 is a flowchart illustrating an example of learning processing. Hereinafter, a procedure for constructing a learned model stored in the storage unit 230 will be described with reference to FIGS. 5 and 6, taking a case of constructing a learned model for estimating DNA integrity as an example.

Note that the creation procedure illustrated in FIG. 5 may be performed by the microscope system 1 or may be performed by other devices or systems. Furthermore, the learning processing illustrated in FIG. 6 may be performed by a computer different from the image processing device 200, and the image processing device 200 may acquire a completed learned model via a network or via a recording medium. Note that, hereinafter, a case where the microscope system 1 performs the creation procedure illustrated in FIG. 5 and the image processing device 200 performs the learning processing illustrated in FIG. 6 will be described as an example.

The microscope system 1 first photographs a moving image of sperm (Step S1). Here, an operator observes the sperm suspension using the microscope system 1 at, for example, 40 times, and picks up the sperm contained in the sperm suspension one by one. The operator is, for example, an embryo cultivator. Thereafter, the microscope system 1 captures a moving image of each picked-up sperm by, for example, the photographing unit 140. The magnification at this time is, for example, 40 times, 60 times, or the like. In addition, when capturing the moving image, the operator rotates sperm using a tip of a pipette or the like. Thus, the microscope system 1 photographs the entire circumference of the head and neck of the sperm.

Thereafter, the microscope system 1 photographs the moving-imaged sperm again after staining (Step S2). Here, the operator fixes each of the sperm captured in the moving image in Step S1 to a position in the assigned container, and then stains the sperm in the container with a dye that labels DNA damage. Thereafter, the operator observes sperm in the stained container by a fluorescence observation method, and further photographs the sperm with the microscope system 1 to acquire a stained image of the sperm.

The microscope system 1 calculates a DNA fragmentation index (DFI) of each sperm from the stained image acquired in Step S2 (Step S3). The DFI is an index of DNA integrity, and a smaller value is better. A specific method for calculating the DFI is not particularly limited, but for example, as described in Non-Patent Document 2 (McCallum, C. et al., Deep learning-based selection of human sperm with high DNA integrity, Communications Biology 2,250, (2019)), the DFI may be calculated using an Acridine Orange (AO) test.

Finally, the microscope system 1 labels the unstained image of the sperm extracted from the moving image acquired in Step S1 with the DFI calculated in Step S3 (Step S4). Here, the microscope system 1 assigns the DFI to each frame image (unstained image) of the moving image, thereby storing the unstained image of the sperm and the DFI of the sperm in association with each other. Note that the association between the moving image captured in Step S1 and the DFI calculated in Step S3 can be performed by managing the ID assigned to the moving image and the position in the container.

In FIG. 6, learning processing is performed by using a large number of unstained images labeled by the procedure of FIG. 5 as a learning data set. When a sufficient amount of learning data set is prepared by the procedure of FIG. 5, the image processing device 200 first acquires the created learning data set (Step S11).

Thereafter, the image processing device 200 causes a model that performs integrity estimation for estimating DNA integrity for sperm to learn (Step S12). Here, the image processing device 200 trains, verifies, and tests the model using the learning data set. Note that the learning data set acquired in Step S11 may be divided into training data and verification data and test data in Step S12. In that case, it is desirable that the training data and verification data is used for both training and verification using cross validation (cross verification). Note that the training data is data used for training the model, and the verification data is data used for verification of the model. The test data is data used for testing the model.

The image processing device 200 repeats the above processing until the model clears the test, and when the model is cleared, that is, when the learning ends (Step S13 YES), the processing of FIG. 6 ends. By performing the processing of FIG. 6, a learned model that performs sufficiently trained integrity estimation can be obtained. The obtained learned model is stored in the storage unit 230 of the image processing device 200. Note that this learned model is an example of a learned model in which parameters are adjusted by supervised learning using, as training data, a data set including an unstained image obtained by photographing sperm before staining and a label attached to the unstained image and indicating DNA integrity calculated from a stained image obtained by photographing sperm after staining.

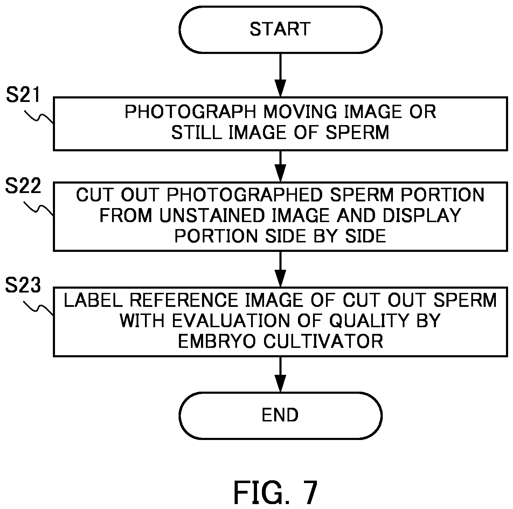

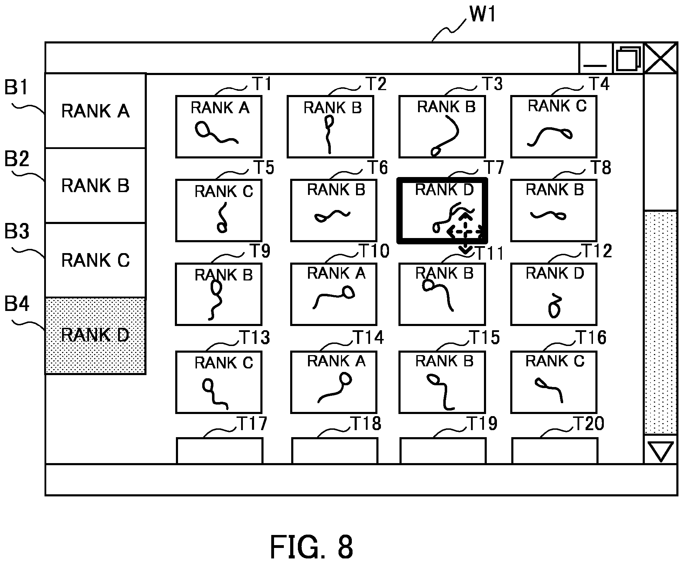

FIG. 7 is a flowchart illustrating another example of the procedure of creating the learning data set. FIG. 8 is a diagram illustrating an example of a screen for creating a learning data set. In FIGS. 5 and 6, learning of a model for performing integrity estimation for estimating DNA integrity for sperm has been described as an example. However, in a case where a model for performing quality estimation for estimating sperm morphological quality and sperm motility quality is learned, a learning data set may be created by a creation procedure illustrated in FIG. 7 instead of the creation procedure illustrated in FIG. 5. Hereinafter, with reference to FIGS. 7 and 8, a procedure for constructing a learned model stored in the storage unit 230 will be described by taking a case of constructing a learned model for estimating at least one quality of sperm morphology and motility as an example.

First, the microscope system 1 photographs a moving image or a still image of sperm (Step S21). Here, the operator observes the sperm suspension using the microscope system 1 at, for example, 20 times, and photographs the sperm in the sperm suspension as a moving image or a still image by the photographing unit 140.

Next, the microscope system 1 cuts out an image of the sperm portion from the photographed image (moving image or still image) and displays the images side by side (Step S22). Here, the microscope system 1 reads the moving image or the still image captured in Step S21, cuts out an image of the sperm portion from the moving image or the still image, and displays the images side by side on the display device 30 as illustrated in FIG. 8. The operator evaluates the sperm morphological quality and sperm motility quality from the sperm images displayed side by side. For this reason, it is desirable that the operator is a skilled embryo cultivator having a high fertilization success rate. Hereinafter, the image obtained by cutting out the sperm portion is referred to as a reference image.

As shown in Figure 8, once each of the sperm reference images has been evaluated by the embryo cultivator, the microscope system 1 labels each reference image with an evaluation of quality by the embryo cultivator (Step S23). Here, the microscope system 1 assigns a quality rank to the reference image (unstained image) of each sperm, thereby storing the unstained image of the sperm and the quality of the sperm in association with each other.

Note that, in the example of FIG. 8, the image (T1, T10, T14,...) clicked in a state where the button B1 on the window W1 is selected is saved in association with the label of the rank A indicating the quality. In addition, the image (T2, T3, T6, T8, T9, T11, T15...) clicked in a state where the button B2 is selected is stored in association with the label of the rank B indicating the quality. In addition, the image (T4, T5, T13, T16...) clicked in a state where the button B3 is selected is stored in association with the label of the rank C indicating the quality. In addition, the image (T7, T12...) clicked in a state where the button B4 is selected is stored in association with the label of the rank D indicating the quality. Note that the rank A is a rank given to an image evaluated to have the highest quality, and the rank D is given to an image evaluated to have the lowest quality. That is, the ranks A, B, C, and D indicate that the quality decreases in this order.

The learning processing illustrated in FIG. 6 may be performed by using a large number of unstained images labeled in the procedure of FIG. 7 as a learning data set. As a result, it is possible to obtain a learned model that performs quality estimation for estimating the morphological quality of sperm and the quality of sperm motility by the learning processing illustrated in FIG. 6. The obtained learned model is stored in the storage unit 230 of the image processing device 200. Note that this learned model is a learned model in which parameters are adjusted by supervised learning using, as training data, a data set including a reference image obtained by photographing sperm and a label that is attached to the reference image and indicates sperm quality evaluated by an embryo cultivator on the basis of the reference image.

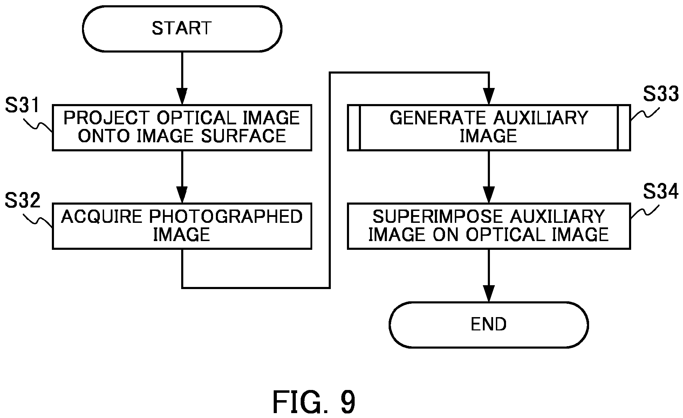

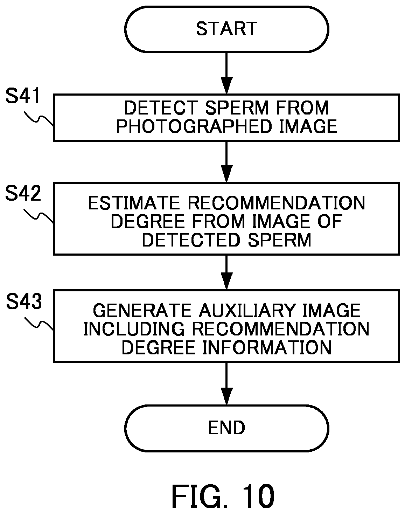

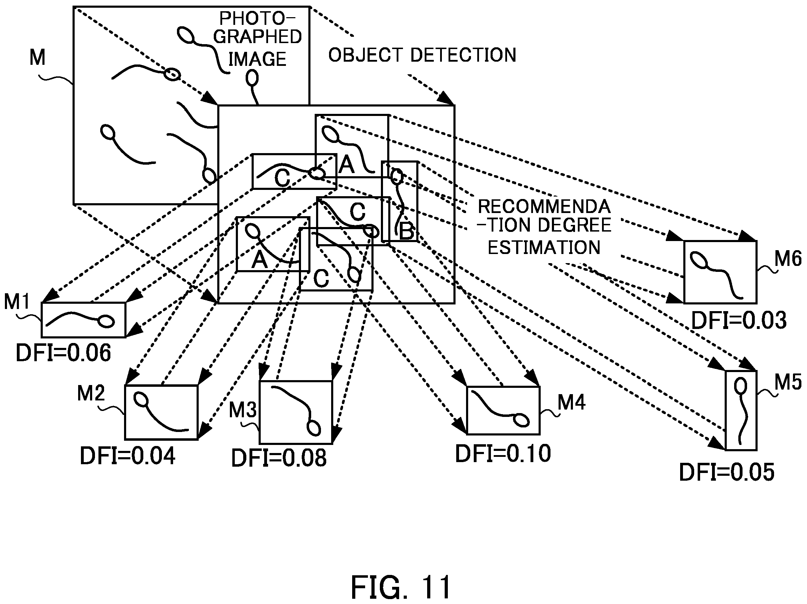

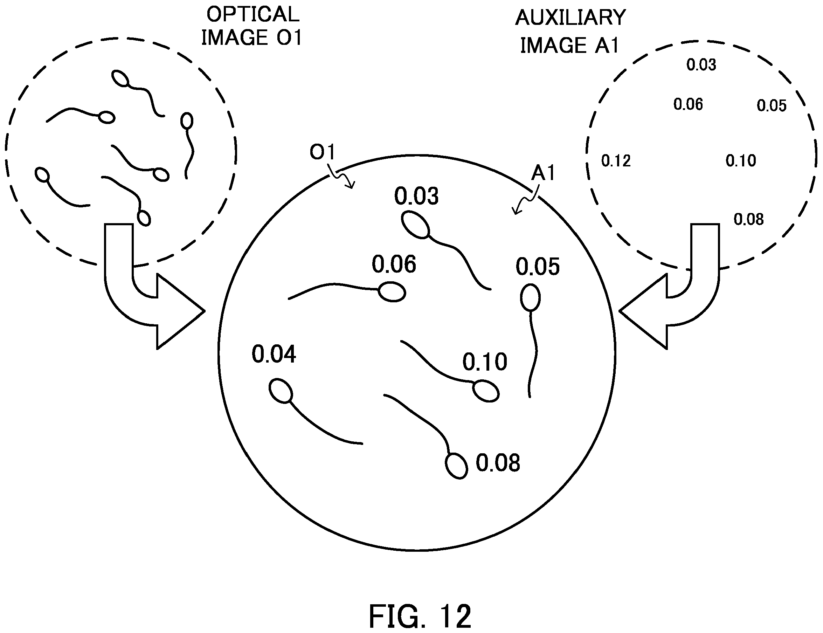

FIG. 9 is a flowchart illustrating an example of sperm sorting support processing. FIG. 10 is a flowchart illustrating an example of the auxiliary image generation processing. FIG. 11 is a diagram for explaining image processing on a photographed image. FIG. 12 is a diagram for describing an image projected on an image surface. Hereinafter, a sperm sorting support method performed in the microscope system 1 by using the learned model obtained by the above-described learning processing will be described with reference to FIGS. 9 to 12.

When the sperm sorting support processing illustrated in FIG. 9 is started, the microscope system 1 first projects an optical image onto an image surface (Step S31). Here, an optical system including an objective 102 and an tube lens 103 forms an optical image O1 of the sperm suspension, for example, as illustrated in FIG. 12, on an optical path of the microscope 100 based on light from the sperm suspension that is a sample placed on a stage of the microscope 100.

At the same time as Step S31, the microscope system 1 acquires a photographed image (Step S32). Here, the photographing device 143 obtains a photographed image of the sperm suspension based on light from the sperm suspension and outputs the photographed image to the image processing device 200.

Thereafter, the microscope system 1 performs auxiliary image generation processing illustrated in FIG. 10 (Step S33). In the auxiliary image generation processing, the image processing device 200 first detects sperm from the photographed image (Step S41). Here, for example, as illustrated in FIG. 11, the image analysis unit 210 inputs the photographed image M to the learned model as an input image, thereby performing object detection for sperm. At this time, as illustrated in FIG. 11, the image analysis unit 210 may classify and detect sperm by the morphological or motile quality of sperm. Note that FIG. 11 illustrates a state in which sperm detected by object detection are classified from rank A to rank C. In this case, the object detection in Step S41 may be regarded as also serving as the recommendation degree estimation in Step S42 described later.

Furthermore, the image processing device 200 estimates the recommendation degree from the image of the sperm detected in Step S41 (Step S42). Here, for example, as illustrated in FIG. 11, the image analysis unit 210 inputs an image (images M1 to M6) of detected sperm to the learned model as an input image, thereby estimating the recommendation degree of sperm. Note that FIG. 11 illustrates an example in which DFI is calculated as the recommendation degree.

Thereafter, the image processing device 200 generates an auxiliary image including sperm recommendation degree information (Step S43). Here, the image generation unit 220 generates recommendation degree information on the recommendation degree of sperm on the basis of the result of the recommendation degree estimation in Step S42, and generates an auxiliary image A1 including the recommendation degree information as illustrated in FIG. 12, for example.

When the auxiliary image generation processing is ended, the microscope system 1 superimposes the auxiliary image on the optical image (Step S34), and ends the processing illustrated in FIG. 9. Here, the projection device 153 projects the auxiliary image A1 generated in Step S33 onto the image surface IP on which the optical image O1 is formed, thereby superimposing the auxiliary image A1 on the optical image O1 as illustrated in FIG. 12.

As a result, the embryo cultivator who is the user of the microscope system 1 can sort the sperm while viewing the image in which the optical image O1 including the sperm and the auxiliary image A1 including the auxiliary information contributing to the sperm sorting are superimposed as illustrated in FIG. 12 by looking through the eyepiece 101. Therefore, according to the microscope system 1, it is possible to support sorting operation of sperm by an embryo cultivator.

In addition, since the recommendation degree information of sperm is included in the auxiliary information, it is easy to determine good sperm, and it is possible to quickly perform the sperm sorting operation in a short time. Furthermore, by including the integrity information on the DNA integrity of sperm as the recommendation degree information, it is possible to sort sperm in consideration of the presence or absence of DNA damage that is difficult to determine from only the optical image O1. Therefore, according to the microscope system 1, the embryo cultivator can sort good sperm with higher reliability than determination based on the conventional form and motility.

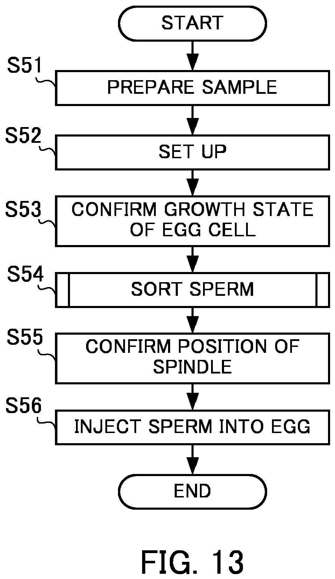

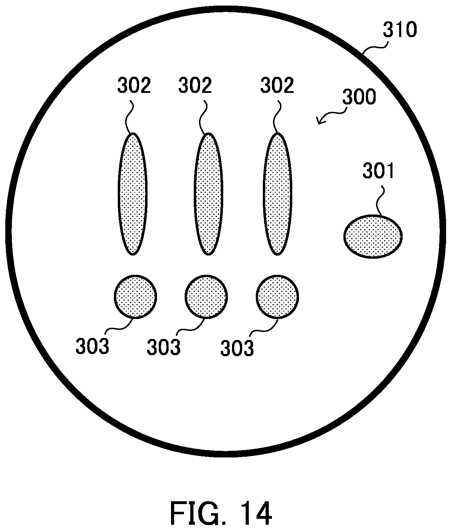

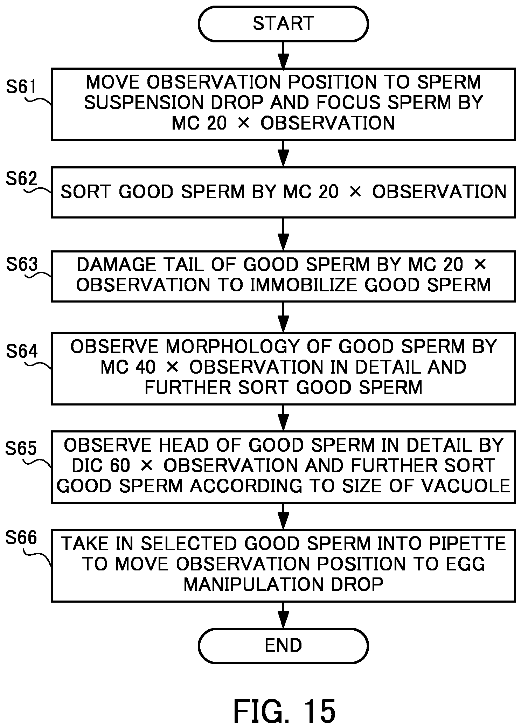

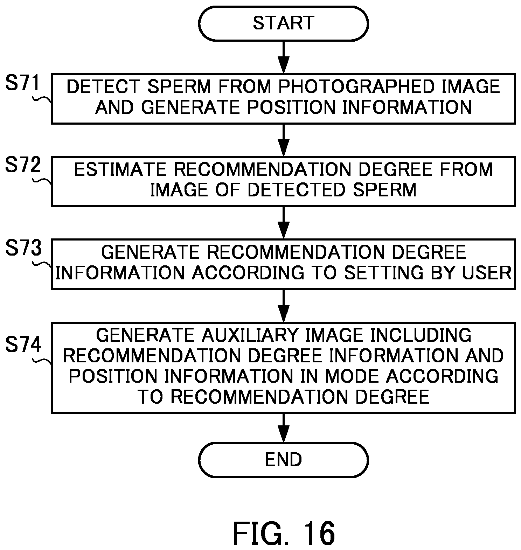

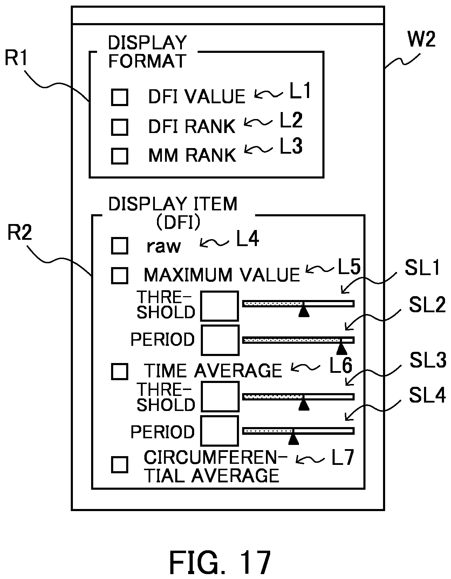

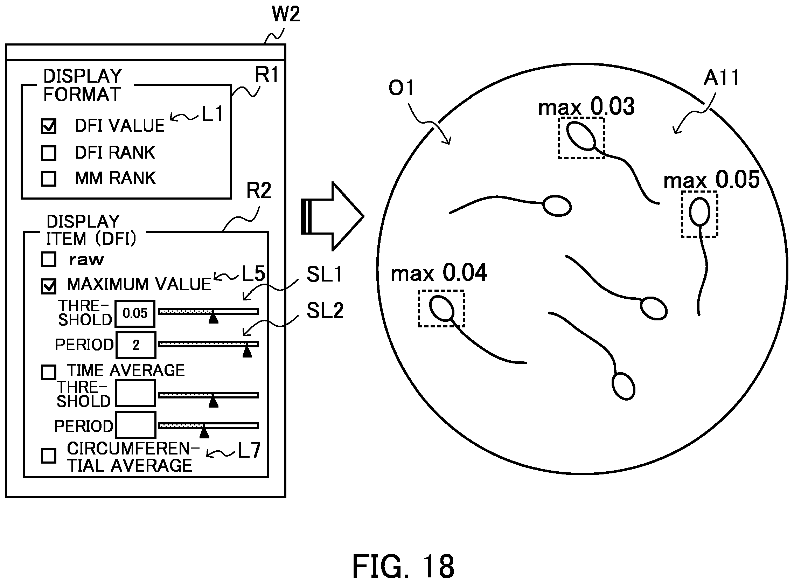

FIG. 13 is a flowchart illustrating an example of the procedure of the ICSI. FIG. 14 is a diagram illustrating a configuration of a drop formed as the sample 300 in the Petri dish 310. FIG. 15 is a flowchart illustrating an example of a sperm sorting procedure. FIG. 16 is a flowchart illustrating another example of the auxiliary image generation processing. FIG. 17 is a diagram for describing a configuration example of an auxiliary image setting window. FIGS. 18 to 24 are diagrams illustrating a relationship between the auxiliary image setting and the image viewed from eyepiece 101. Hereinafter, specific utilization of the sperm sorting support method performed by the microscope system 1 in the ICSI will be described with reference to FIGS. 13 to 24.

First, the user prepares a sample (Step S51). Here, for example, as illustrated in FIG. 14, the user creates a sample 300 including a plurality of drops in the Petri dish 310 and disposes the sample on the stage 111.

The drop 301 is a drop for cleaning, and is used for cleaning a pipette. The drop 302 is a sperm suspension drop, for example, obtained by dropping a sperm suspension into a PVP solution. The drop 303 is an egg manipulation drop, and is, for example, an egg put in an m-HTF solution. The m-HTF solution is an Hepps-containing HTF solution to which 10% serum is added. These drops are covered with mineral oil.

Next, the user sets up the microscope system 1 (Step S52). Here, for example, the user presses the button 51 of the input device 50 to switch the setting of the microscope system 1 to BF4 × observation. Thereafter, the input device 40 is operated to adjust the positions of the pipette 43 and the pipette 44, and the pipette 43 and the pipette 44 are focused. Further, the stage 111 is moved to wash the pipette 43 and the pipette 44 with the drop 301 (cleaning drop).

When the setup is completed, the user confirms the growth state of the egg (egg cell) in the drop 303 (egg manipulation drop) (Step S53). Here, for example, the user presses the button 53 of the input device 50 to switch the setting of the microscope system 1 to MC 20 × observation. The morphology of the egg is observed by MC 20 × observation, and the egg is sorted. Further, for example, the button 55 of the input device 50 may be pressed to switch the setting of the microscope system 1 to PO 20 × observation. By observing the spindle of the egg by PO 20 × observation, a degree of maturation of the egg may be determined, and the egg may be further sorted.

When the sorting of the egg is completed, the user sorts sperm according to the procedure illustrated in FIG. 15 (Step S54). First, for example, the user presses the button 53 of the input device 50 to switch the setting of the microscope system 1 to MC 20 × observation. Then, the observation position is moved to the drop 302 (sperm suspension drop) by moving the stage 111, and the sperm is focused by MC 20 × observation (Step S61).

Next, the user sorts good sperm suitable for fertilization by MC 20 × observation (Step S62). Conventionally, in this step, an embryo cultivator determines sperm quality based on sperm morphology and motility observed in an optical image, and sorts good sperm based on the determination. However, criteria for determining good sperm are not necessarily clear. For this reason, the sperm is often selected depending on the experience and intuition of the embryo cultivator, and as a result, the determination differs depending on the embryo cultivator. This has caused a difference in the fertilization success rate among embryo cultivators. In view of such a problem, the microscope system 1 supports the sperm sorting operation of the embryo cultivator by superimposing an auxiliary image including auxiliary information contributing to sperm sorting on the optical image.

Specifically, in Step S62, the microscope system 1 performs the auxiliary image generation processing illustrated in FIG. 16, and projects the generated auxiliary image on the image surface as a result. First, the image processing device 200 detects sperm from the photographed image of the drop 302 (sperm suspension drop) captured by the photographing device 143, and generates position information on the position of the detected sperm (Step S71). Here, for example, as described above, object detection for sperm is performed using the learned model.

Furthermore, the image processing device 200 estimates the recommendation degree from the image of the sperm detected in the photographed image (Step S72). Here, for example, as described above, recommendation degree estimation such as integrity estimation and quality estimation is performed using the learned model. Note that, hereinafter, in Step S72, a case where the DFI is estimated for each sperm by integrity estimation, and the rank (hereinafter, it is referred to as MM rank.) of the morphology and the motility of sperm is estimated for each sperm by quality estimation will be described as an example.

Next, the image processing device 200 generates the recommendation degree information according to the setting by the user (Step S73). Here, for example, the image processing device 200 generates the recommendation degree information from the estimation result obtained in Step S72 according to the setting performed in advance on the window W2 illustrated in FIG. 17 displayed on the display device 30 by the user using the input device 60 and the input device 70. Note that the window W2 is an auxiliary image setting window for setting the recommendation degree information included in the auxiliary image, and includes a region R1 for setting a display format and a region R2 for setting a display item.

As illustrated in FIG. 17, the region R1 includes a list box L1 for selecting a DFI value, a list box L2 for selecting a DFI rank, and a list box L3 for selecting a MM rank as display formats of the recommendation degree. Note that the DFI value is the DFI itself, and the DFI rank is obtained by dividing the DFI into predetermined ranges and assigning a rank to each range.

When the user selects the list box L1, the DFI value can be included in the auxiliary image as the recommendation degree information. When the user selects the list box L2, the DFI rank can be included in the auxiliary image as the recommendation degree information. Further, when the user selects the list box L3, the MM rank can be included in the auxiliary image as the recommendation degree information. Note that a plurality of items can be selected from among from the list box L1 to list boxes L3, and in this case, information in a plurality of display formats can be included as the recommendation degree information.

As illustrated in FIG. 17, the region R2 includes, as display items of the recommendation degree (In particular, DFI), a list box L4 for selecting raw, a list box L5 for selecting a maximum value, a list box L6 for selecting a time average, and a list box L7 for selecting a circumferential average. raw is the DFI estimated from the latest photographed image. The maximum value is the maximum value of the DFI estimated within the period set by the slider SL2. The threshold set by the slider SL1 is an upper limit of a maximum value included in the recommendation degree information. The time average is an average value of the DFI estimated within the period set by the slider SL4. The threshold set by the slider SL3 is an upper limit of the average value included in the recommendation degree information. By setting the threshold with the slider SL1 and the slider SL3, it is possible to avoid display of a too high DFI (That is, low evaluation). The circumferential average is an average value of DFI estimated from photographed images captured from various directions by rotating sperm.

When the recommendation degree information is generated, the image processing device 200 generates an auxiliary image including the recommendation degree information and the position information in a mode according to the recommendation degree (Step S74). Here, for example, the image processing device 200 may generate an auxiliary image including the recommendation degree information and the position information in a color according to the recommendation degree. More specifically, in a case where the position information is generated as a bounding box surrounding the sperm, the thickness and line type of the line constituting the bounding box included in the auxiliary image may be varied according to the recommendation degree.

Since the microscope system 1 performs the auxiliary image generation processing illustrated in FIG. 16 and projects the generated auxiliary image on the image surface, in Step S62, the user can sort sperm while viewing the auxiliary image A11 superimposed on the optical image O1 as illustrated in FIG. 18, for example. Note that FIG. 18 illustrates an example of an image projected within the field of view of the eyepiece 101 when the DFI value is selected as the display format and the maximum value is selected as the display item. The displayed DFI is a maximum value for the last 2 seconds and is limited to 0.05 or less by threshold setting.

The sperm sorting in Step S62 is to sort good sperm from a large number of sperm contained in the sperm suspension, and it is expected that a large number of sperm are also contained in the field of view. In such a situation, as shown in FIG. 18, the threshold setting by the slider SL1 is effective. By setting the threshold, only recommendation degree information of sperm having a DFI (here, the maximum value of the DFI) equal to or less than the threshold can be projected, so that the amount of information superimposed on the optical image O1 can be appropriately limited. As a result, it is possible to avoid problems in advance such as a decrease in the visibility of the optical image (sperm) due to projection of the recommendation degree information of a myriad of sperm in the visual field, and conversely, difficulty in understanding necessary information due to too much information. In addition, by limiting the amount of information using the threshold, it is possible for the user to observe only sperm with high evaluation.

Furthermore, in Step S62, since the sperm freely moves around in the sperm suspension, the orientation and shape of the sperm in the photographed image also change from moment to moment. Therefore, even in the same sperm, it is expected that the estimated DFI changes from moment to moment. In such a situation, as illustrated in FIG. 18, the period setting by the slider SL2 is effective. By setting the period, it is possible to extract or reconstruct necessary information from a large number of estimation results obtained within the set period and project the information. This makes it possible to provide the recommendation degree of the sperm to the user without being affected by a temporary estimation result at a specific time.

In FIG. 18, the position information is formed as a bounding box surrounding the head of the sperm. As shown in FIG. 18, the location information may be formed to identify a major portion of the sperm, rather than the entire sperm. Such display of position information is also particularly suitable in Step S62 in which a large number of sperm are expected to be detected.

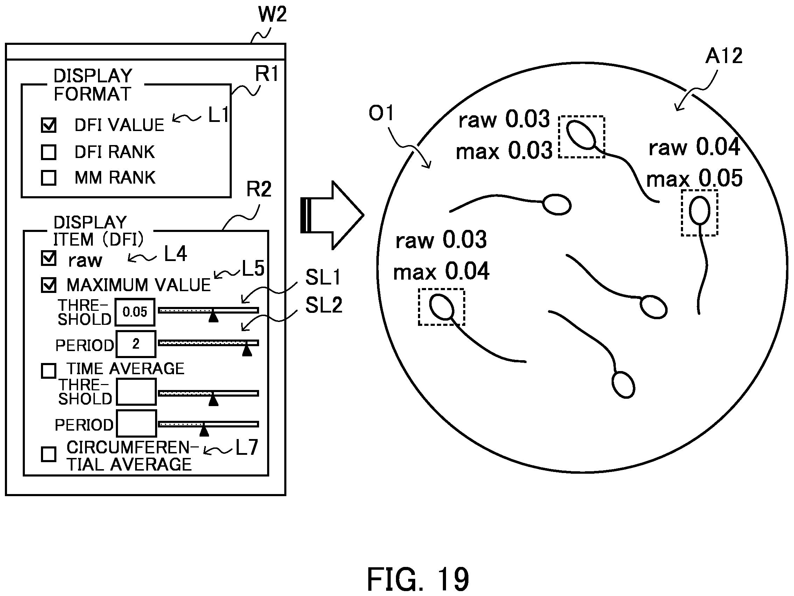

Furthermore, although FIG. 18 illustrates an example in which one piece of recommendation degree information is superimposed on one sperm, a plurality of pieces of recommendation degree information may be superimposed. For example, as illustrated in FIG. 19, by selecting a plurality of display items on the window W2, the auxiliary image A12 including a plurality of pieces of information on DNA integrity (here, raw and the maximum value) for each sperm may be superimposed on the optical image O1. By superimposing a plurality of pieces of information, it is possible to obtain a material that analyzes each sperm in more detail in sperm sorting. Note that FIG. 19 illustrates an example of an image projected within the field of view of the eyepiece 101 when the DFI value is selected as the display format and raw and the maximum value are selected as the display items. The displayed DFI is the latest value (raw) and the maximum value in the last 2 seconds, and is limited to 0.05 or less by the threshold setting.

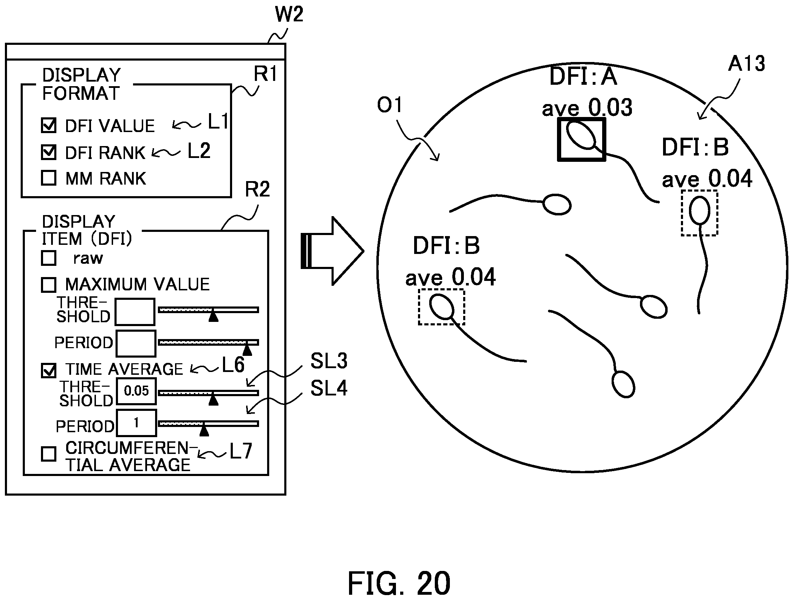

Furthermore, as illustrated in FIG. 20, the auxiliary image A13 including a plurality of pieces of information for each sperm may be superimposed on the optical image O1 by selecting the DFI value and the DFI rank. In this case, since the user hits the sperm to be observed with the DFI rank and can observe the sperm while confirming the DFI value, it is possible to efficiently sort the sperm. Note that FIG. 20 illustrates an example of an image projected within the field of view of the eyepiece 101 when the DFI value and the DFI rank are selected as the display format and the time average is selected as the display item. The DFI displayed together with the DFI rank is a time average value for the most recent 1 second, and is limited to 0.05 or less by threshold setting.

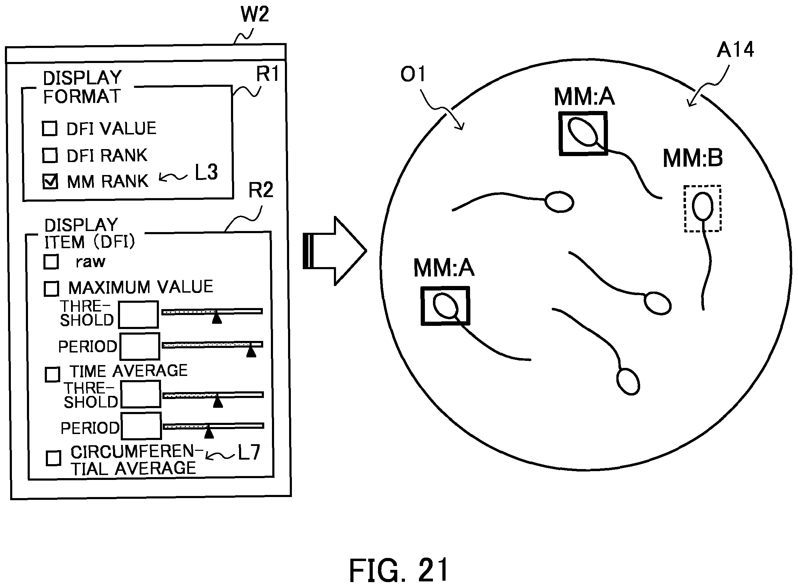

Furthermore, in FIGS. 18 to 20, an example in which the information on DNA integrity is superimposed as the recommendation degree information is illustrated. However, as illustrated in FIG. 21, an auxiliary image A14 including MM rank based on sperm morphology or motility conventionally performed as the recommendation degree information may be superimposed on the optical image O1.

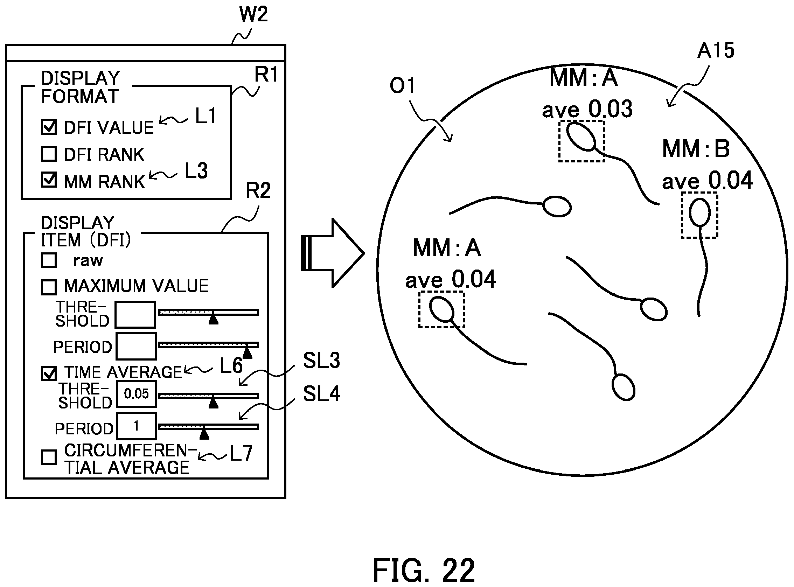

As illustrated in FIG. 22, auxiliary image A15 including both the MM rank and the information on DNA integrity (here, the DFI value) as the recommendation degree information may be superimposed on optical image O1. As a result, the user can sort sperm by combining evaluation based on the same criteria as in the related art and evaluation regarding DNA integrity. Note that FIG. 22 illustrates an example of an image projected within the field of view of the eyepiece 101 when the DFI value and the MM rank are selected as the display format and the time average is selected as the display item. The DFI displayed together with the MM rank is a time average value for the most recent 1 second, and is limited to 0.05 or less by threshold setting.

As described above, in Step S62, since the auxiliary image is superimposed on the optical image O1, the user can easily recognize the sperm to be particularly noted by the auxiliary image, and thus, it is possible to greatly reduce the number of sperm confirmed by the user for sorting good sperm. For this reason, since the sperm sorting operation becomes easy and the sperm sorting can be performed in a short time, the burden of the sorting operation is greatly reduced.

When the good sperm is sorted in Step S62, the user damages the tail of the good sperm by RC 20 × observation to immobilize the good sperm (Step S63). Here, the user immobilizes the good sperm by rubbing the tail of the good sperm against the bottom surface of the Petri dish 310 with a pipette.

Thereafter, the user observes the morphology of the immobilized good sperm in more detail, and further sorts the good sperm (Step S64). Here, for example, the user presses the button 54 of the input device 50 to switch the setting of the microscope system 1 to MC 40 × observation. Thereafter, the user sorts better sperm by MC 40 × observation. Here, similarly to Step S64, the microscope system 1 performs the auxiliary image generation processing illustrated in FIG. 16, and projects the auxiliary image generated as a result on the image surface, thereby supporting the sperm sorting operation of the embryo cultivator.

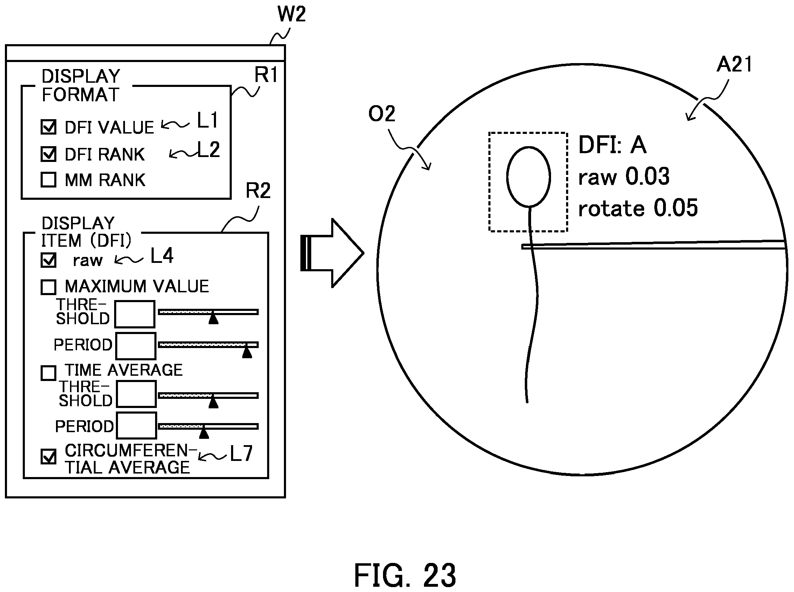

Since the microscope system 1 performs the auxiliary image generation processing illustrated in FIG. 16 and projects the generated auxiliary image on the image surface, in Step S64, the user can sort sperm while viewing the auxiliary image A21 superimposed on the optical image O2 as illustrated in FIG. 23, for example. Note that FIG. 23 illustrates an example of an image projected within the field of view of the eyepiece 101 when the DFI value and the DFI rank are selected as the display format and raw and the circumferential average are selected as the display items.

The sperm sorting in Step S64 is performed with a specific sperm immobilized, and it is expected that a single sperm is included in the field of view. In such a situation, the user can observe the sperm from any direction by adjusting the orientation of the sperm at the tip of the pipette, and the entire circumference of the head and neck of the sperm can be photographed by rolling the sperm at the tip of the pipette. Therefore, as illustrated in FIG. 23, the calculation setting of the circumferential average by the selection of the list box L7 is effective. By calculating the circumferential average, it is possible to provide the user with highly reliable DNA integrity regardless of the imaging direction.

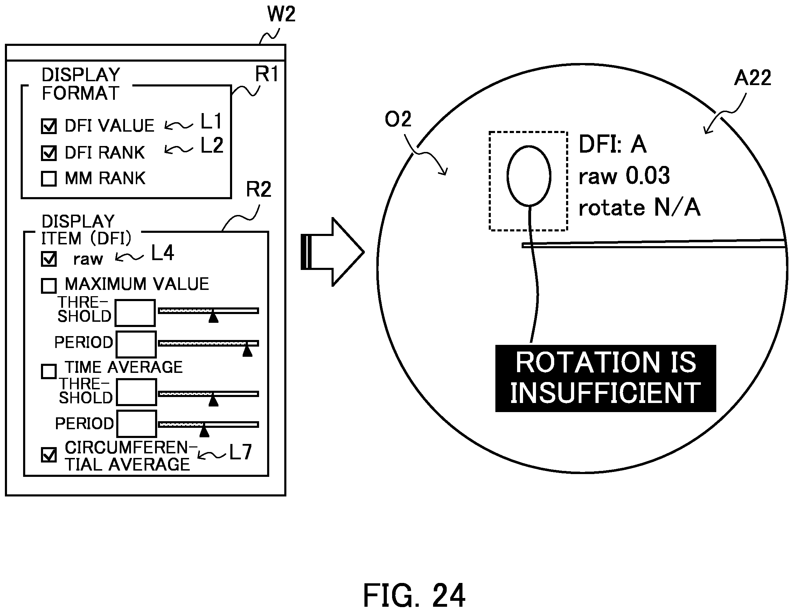

Note that, in a case where the rotation of the sperm is insufficient, the direction dependence is large even if the circumferential average is calculated, and highly reliable information cannot be provided. Therefore, in Step S64, the image processing device 200 may calculate the rotation amount of the sperm, and in a case where the rotation amount is insufficient (for example, the rotation amount is less than 360°), as illustrated in FIG. 24, may generate an auxiliary image A22 including information for notifying insufficient rotation of the sperm, and the projection device 153 may superimpose the auxiliary image A22 on the optical image O2. That is, the image analysis performed by the image analysis unit 210 may include processing of estimating the recommended operation in addition to the object detection and the recommendation degree estimation, and the image analysis unit 210 may estimate the recommended operation to be performed during the sperm sorting on the basis of the photographed image. The notification of the recommended operation may be notified by voice, light, vibration, or the like instead of or in addition to visually notifying using the auxiliary image. That is, the notification device may be the projection device 153, or may be a speaker, a lamp, a vibrator, or the like (not illustrated).

When the sorting of the good sperm in the MC 40 × observation is completed, the user further observes the head of the good sperm in detail, and further sorts the good sperm according to the size of the vacuole present in the head (Step S65). Here, for example, the user presses the button 56 of the input device 50 to switch the setting of the microscope system 1 to DIC 60 × observation. Thereafter, the user sorts good sperm having small vacuoles as better sperm by DIC 60 × observation. Step S65 may be performed under MC 40 × observation. In this case, the user recognizes a bright spot generated on the head as a vacuole, thereby sorting good sperm.

Thereafter, the user takes in the selected good sperm into the pipette 44 that is an injection pipette, moves the observation position to the drop 303 (egg manipulation drop) (Step S66), and ends the series of procedures of sperm sorting shown in FIG. 15.

When the sperm sorting is completed, the user confirms the position of the spindle to prepare for injection of good sperm (Step S55). Here, the user observes the egg selected in Step S53 present in the drop 303 and confirms the position of the spindle of the egg. Specifically, for example, the user presses the button 55 of the input device 50 to switch the setting of the microscope system 1 to PO 20 × observation. Thereafter, the user operates the pipette 43, which is a holding pipette, so that the spindle of the egg visualized by the PO 20 × observation is positioned in the 12:00 or 6:00 direction, thereby changing the orientation of the spindle. This is to prevent the spindle from being damaged by a pipette that is thrust against the egg from the 3:00 or 9:00 direction in Step S56 to be described later.

Finally, the user injects sperm into the egg (Step S56), and ends the ICSI. Here, for example, the user presses the button 53 of the input device 50 to switch the setting of the microscope system 1 to MC 20 × observation. Thereafter, in the MC 20 × observation, the user fixes the egg whose orientation has been adjusted in Step S55 with the pipette 43, which is a holding pipette, and pierces the pipette 44, which is an injection pipette. Thereafter, good sperm is injected into the inside of the egg from the pipette 44.

When the series of ICSI procedures illustrated in FIG. 13 is completed, the user returns the egg into which the sperm has been injected to the incubator and cultures the egg. In addition, the user may operate the processing device 200 using the input device 60 and the input device 70 to store the information obtained by the ICSI in the database server 20. For example, the image data of the egg into which the sperm is injected, the image data of the selected good sperm, the ICSI working time, and the like may be stored in the database server 20 in association with the patient information (maternal clinical data, semen test results including sperm, or the like) of the sperm and the egg and the data of the culture solution (for example, type, concentration, PH, or the like) of the sperm and the egg.

As described above, in the microscope system 1, the auxiliary image contributing to the sperm sorting is projected on the image surface in the ICSI. The size of the sperm is about 60 um, and the objective of at least 20 times is used to distinguish good sperm. In general, since the number of fields of view of the inverted microscope is about 22, the actual field of view is about Φ1 mm. The task of sorting sperm freely moving around within this actual field of view Φ1mm is a very difficult task. In general, since sperm estimated to be good sperm are highly motile and the ICSI operation needs to be performed in a short time, in the sperm sorting operation, it is necessary to quickly observe the form of sperm moving relatively fast to determine quality. The superimposition of the auxiliary image contributing to the good sperm sorting on the optical image in an operation environment where such severe restrictions are imposed greatly contributes to the reduction of the burden of the sperm sorting operation. In addition, the improvement of the fertilization success rate and the suppression of the variation in the fertilization success rate among embryo cultivators can be achieved at the same time by incorporating the knowledge of a skilled embryo cultivator as an analysis algorithm in the analysis (recommendation degree estimation) for specifying sperm to be noted. In particular, it is possible to sort sperm with higher reliability than before by including, in the auxiliary image, information on DNA integrity that cannot be distinguished even by a skilled embryo cultivator. Therefore, according to the microscope system 1, it is possible to effectively assist sperm sorting by the user.

The above-described embodiments illustrate specific examples for facilitating understanding of the invention, and the embodiments of the present invention are not limited thereto. The microscope system, the projection unit, and the sperm sorting support method can be variously modified and changed without departing from the scope of the claims.

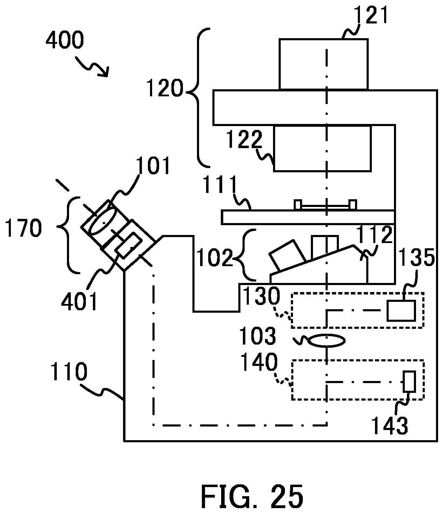

FIG. 25 is a diagram illustrating a configuration of the microscope 400. In the above-described embodiment, the projection device 153 arranged on the optical path branched from the optical path between the objective 102 and the eyepiece 101 has been exemplified, but the device that superimposes the auxiliary image may be arranged on the optical path between the objective 102 and the eyepiece 101 as illustrated in FIG. 25. The microscope 400 is different from the microscope 100 in that a display device 401 is included on an image surface on which an optical image is formed instead of the projection unit 150. The display device 401 is a transmissive image display device, and is an example of a superimposition device that superimposes an auxiliary image on an optical image by directly displaying the auxiliary image on an image surface. Even in the case of using the microscope 400, it is possible to support sperm sorting of the embryo cultivator as in the case of using the microscope 100.

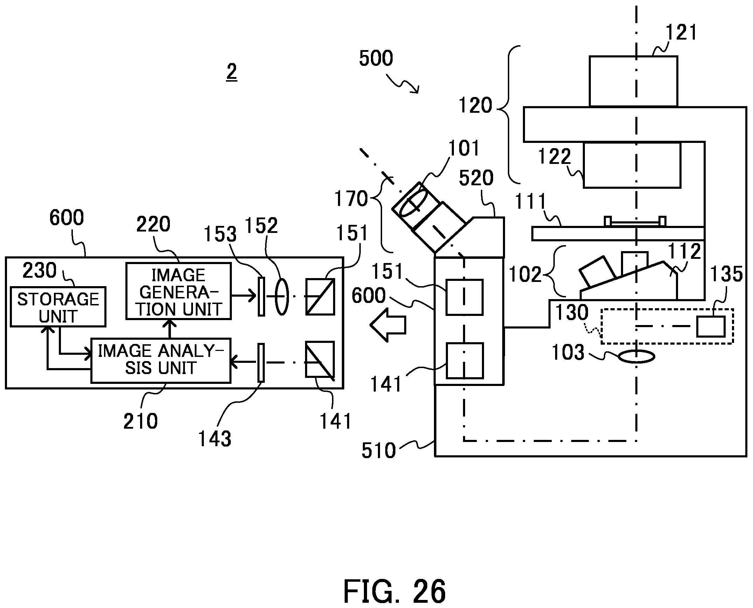

FIG. 26 is a diagram illustrating a configuration of the microscope system 2. In the above-described embodiment, an example has been described in which the photographing device 143 and the projection device 153 are provided in the microscope 100, and the image processing device 200 independent of the microscope 100 exchanges data with the photographing device 143 and the projection device 153 to superimpose the auxiliary image on the optical image. However, the photographing device, the projection device, and the image processing device may be integrally configured as illustrated in FIG. 26.

As illustrated in FIG. 26, a projection unit 600 mounted between a microscope body 510 and a lens barrel 520 of a microscope 500 may include the photographing device 143, the projection device 153, and the image processing device (image analysis unit 210, image generation unit 220, and storage unit 230). By using the projection unit 600, it is possible to easily add a function of superimposing an auxiliary image on an existing microscope.

In the above description, the DFI value, the DFI rank, and the MM rank are exemplified as the recommendation degree information included in the auxiliary image, but the recommendation degree information is not limited thereto. For example, the recommendation degree information may be a sperm quality determination result. In this case, the object detection and the recommendation degree estimation may be performed using the learned model in which the quality of the sperm is learned.

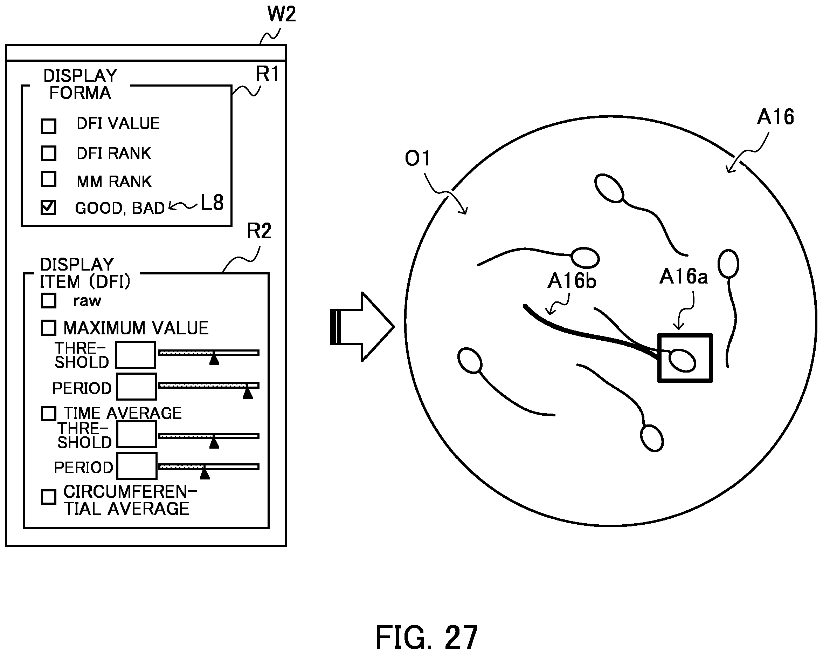

FIG. 27 is a diagram illustrating still another example of the relationship between the auxiliary image setting and the image viewed from eyepiece 101. As illustrated in FIG. 27, the auxiliary image A16 including the quality determination result may be superimposed on the optical image O1 by selecting the list box L8 provided in the region R1 for selecting the display format of the recommendation degree.

For example, the auxiliary image A16 may include a bounding box A16a surrounding the sperm having the largest number of times of being determined to be "good" by the quality determination performed for each frame. Alternatively, the sperm having the largest value calculated by multiplying the number of times determined as "good" by the value of the VSL (linear velocity) may be surrounded by the bounding box A16a. In particular, in a case where the quality determination is performed based on the morphological characteristics of the sperm, the motility of the sperm may not be sufficiently considered. Therefore, it is desirable to generate the auxiliary image based on a value calculated by multiplying the VSL by the number of times of determination as "good". Furthermore, in order to consider motility, a movement trajectory A16b of a sperm surrounded by a bounding box A16a may be included in the auxiliary image A16.

As illustrated in FIG. 27, since only the most highly evaluated sperm is surrounded by the bounding box, the user can grasp the sperm to be noted at a glance.

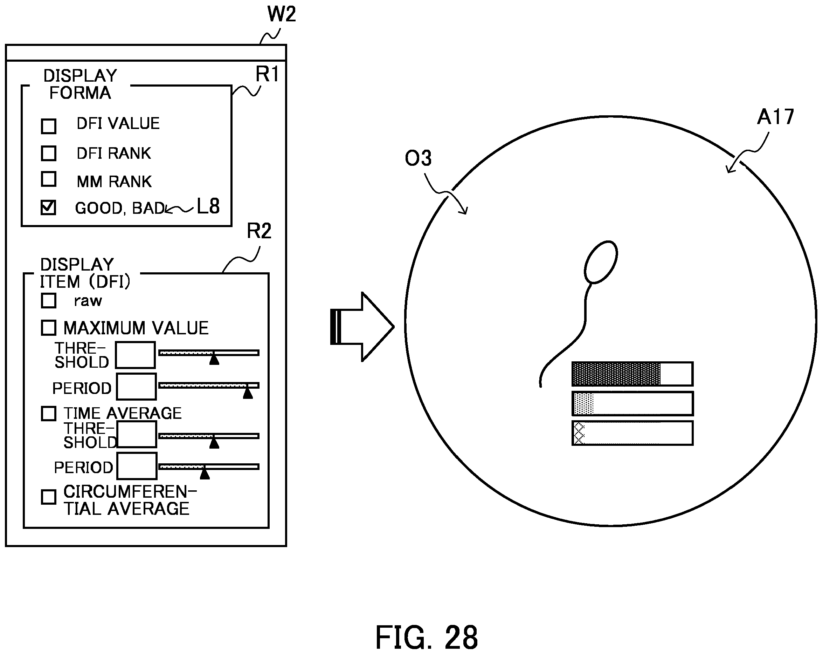

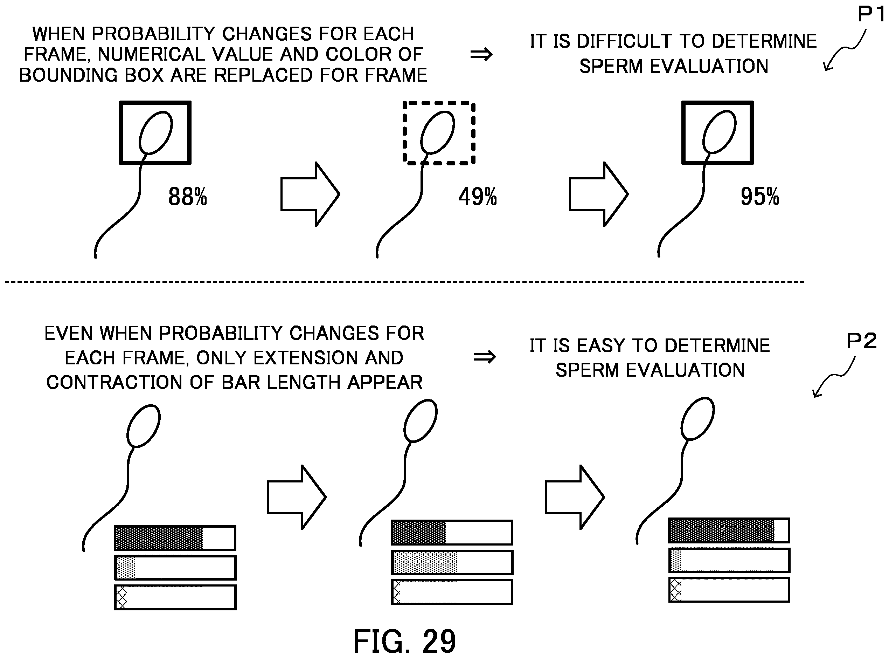

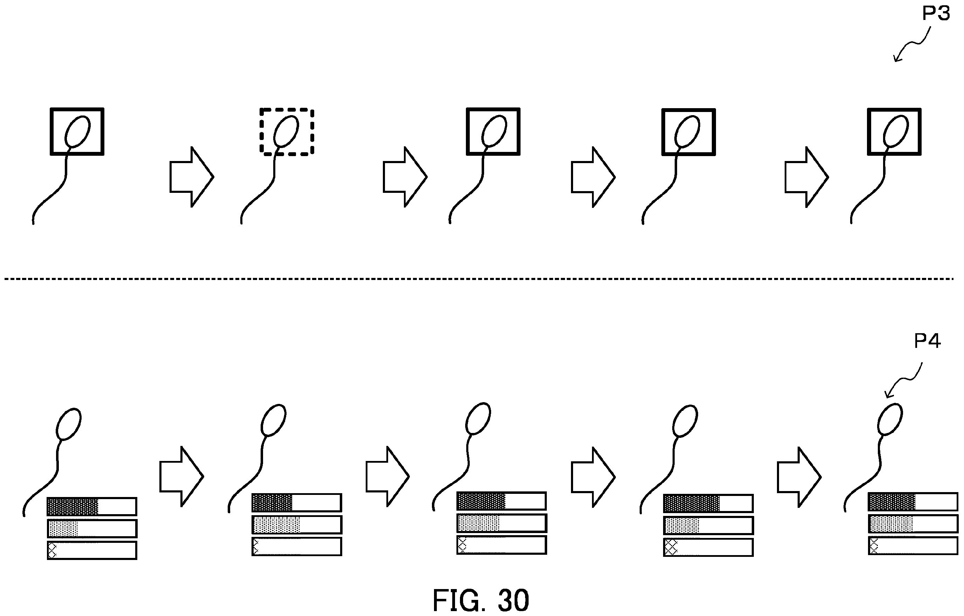

FIG. 28 is a diagram illustrating still another example of the relationship between the auxiliary image setting and the image viewed from eyepiece 101. FIGS. 29 and 30 are diagrams for explaining advantages of the bar display. As illustrated in FIG. 28, an auxiliary image A17 including a bar indicating the quality determination result may be superimposed on the optical image O3 by selecting the list box L8 provided in the region R1 for selecting the display format of the recommendation degree. That is, the recommendation degree may be presented to the user by bar display.

Note that the three bars indicating the quality determination results illustrated in FIG. 28 indicate, by length, the probabilities of the respective classifications "good", "bad", and "impossible to determine" output from the learned model, respectively.

As indicated by a pattern P1 in FIG. 29, in a case where the result of the quality determination performed for each frame is displayed using the numerical value indicating the probability and the color of the bounding box, when the probability changes for each frame, the numerical value and the color change for each frame. When the numerical value or the color changes in a short period such as changing from frame to frame, it is difficult to correctly read the contents. Meanwhile, as indicated by a pattern P2, in the case of the bar display, the change appears as extension and contraction of the length, and thus, even when the length changes in a short cycle, whether the probability is high or low can be easily determined.

Furthermore, as indicated by a pattern P3 in FIG. 30, in a case where the result of the quality determination performed for each frame is displayed using the color of the bounding box, the determination result is presented to the user as simplified information indicating only the classification result. Such a display is very simple, and thus is suitable for applications such as making a hit against good sperm candidates used for microinsemination among a large number of sperm. Meanwhile, it is not possible for the user to recognize the probability of being classified into "good" or "bad". For this reason, the user is not aware of the possibility that sperm classified as "good" is "bad", and is unable to select a better option even if there is a better option. Meanwhile, as illustrated in a pattern P4 of FIG. 30, in the case of the bar display, it can be noticed that there is no large difference between the probability of "good" and the probability of "bad". Thus, even when the determination of "good" prevails over the determination of "bad", the user can continue to search for better sperm without selecting that sperm. Therefore, by using the bar display, the user can reliably select good sperm.

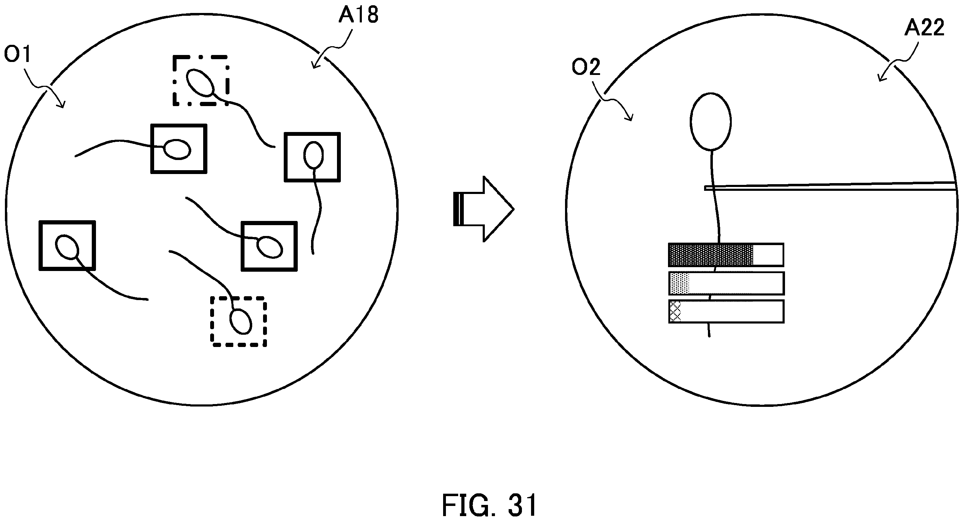

FIG. 31 is a diagram for describing a desirable modification of the display format of the auxiliary image. As described above, the simple display using the bounding box and the bar display have advantages, and it is possible to efficiently and reliably select good sperm by selectively using them according to the situation. Specifically, for example, in a case where good sperm is selected from a plurality of sperm, such as the processing in Step S62 in FIG. 15, as illustrated in FIG. 31, an auxiliary image A17 including a bounding box representing the quality determination result of each sperm in color may be superimposed on the optical image O1. Furthermore, for example, in a case where the immobilized sperm is observed in detail, such as the processing in Step S64 and Step S65 in FIG. 15, as illustrated in FIG. 31, an auxiliary image A22 representing the immobilized sperm quality determination result using a plurality of bars may be superimposed on the optical image O2.

### Reference Signs List

- 1, 2: microscope system
- 30: display device
- 100, 400, 500: microscope
- 101: eyepiece
- 102, 102a, 102b, 102c: objective
- 103: tube lens
- 111: stage
- 143: photographing device
- 153: projection device
- 200: image processing device
- 201: processor
- 202: storage device
- 210: image analysis unit
- 220: image generation unit
- 230: storage unit
- 300: sample
- 401: display device
- 600: projection unit

## Claims

1. A microscope system comprising:
a microscope;
a photographing device that acquires a photographed image of a sample containing sperm placed on a stage of the microscope;
an image processing device that generates an auxiliary image including auxiliary information contributing to sperm sorting on the basis of the photographed image acquired by the photographing device; and
a superimposition device that superimposes the auxiliary image generated by the image processing device on an optical image of the sample formed on an image surface on an optical path of the microscope.

2. The microscope system according to claim 1,
wherein the image processing device includes
an image analysis unit that performs image analysis including object detection for the sperm with respect to the photographed image and recommendation degree estimation for estimating a recommendation degree of the sperm detected in the object detection, and
an image generation unit that generates the auxiliary image based on a result of the image analysis, and
the auxiliary information includes recommendation degree information on the recommendation degree of the sperm, the recommendation degree information being generated on the basis of a result of the recommendation degree estimation.

3. The microscope system according to claim 2,
wherein at least a part of the recommendation degree information is calculated based on a history of a result of the recommendation degree estimation within a predetermined period.

4. The microscope system according to claim 2 or 3,
wherein the recommendation degree estimation includes integrity estimation that estimates DNA integrity of the sperm detected in the object detection, and
the recommendation degree information includes integrity information on the DNA integrity of the sperm, the integrity information being generated based on a result of the integrity estimation.

5. The microscope system according to claim 4,
wherein the image processing device further includes a storage unit that stores a learned model in which the DNA integrity of the sperm with respect to sperm morphology has been learned, and
the image analysis unit performs the integrity estimation using the learned model stored in the storage unit.

6. The microscope system according to claim 5,
wherein the learned model is a learned model in which a parameter is adjusted by supervised learning using a data set including an unstained image obtained by photographing the sperm before staining and a label attached to the unstained image and indicating DNA integrity calculated from a stained image obtained by photographing sperm after staining as training data.

7. The microscope system according to any one of claims 4 to 6,
wherein the integrity information includes at least one of a DNA Fragmentation Index (DFI) indicating the DNA integrity of the sperm and a DFI rank according to the DFI.

8. The microscope system according to claim 7,
wherein the DFI or the DFI rank includes at least two of latest information, average information, and maximum information.

9. The microscope system according to claim 2 or 3,
wherein the recommendation degree estimation includes a quality estimation that estimates a quality of at least one of a sperm morphology and sperm motility detected in the object detection, and
the recommendation degree information includes quality information on at least one of a sperm morphological quality and a sperm motility quality, the quality information being generated on the basis of a result of the quality estimation.

10. The microscope system according to claim 9,
wherein the image processing device further includes a storage unit that stores a learned model in which sperm quality with respect to at least one of sperm morphology and sperm motility has been learned, and
the image analysis unit performs the quality estimation using the learned model stored in the storage unit.

11. The microscope system according to claim 10,
wherein the learned model is a learned model in which a parameter is adjusted by supervised learning using, as training data, a data set including a reference image obtained by photographing the sperm and a label attached to the reference image and indicating sperm quality evaluated by an embryo cultivator on the basis of the reference image.

12. The microscope system according to any one of claims 2 to 11,
wherein the recommendation degree information has a mode according to a recommendation degree estimated by the recommendation degree estimation.

13. The microscope system according to claim 12,
wherein the recommendation degree information includes a color corresponding to the recommendation degree estimated by the recommendation degree estimation.

14. The microscope system according to any one of claims 2 to 13,
wherein the auxiliary information further includes position information on a position of the sperm generated on the basis of a result of the object detection.

15. The microscope system according to claim 14,
wherein the position information has a mode according to a recommendation degree estimated by the recommendation degree estimation.

16. The microscope system according to claim 15,
wherein the position information has a color corresponding to the recommendation degree estimated by the recommendation degree estimation.

17. The microscope system according to any one of claims 2 to 16,
wherein the image analysis further includes processing of estimating a recommended operation to be performed during sperm sorting based on the photographed image, and
the microscope system further includes a notification device that notifies an estimated recommended operation.

18. The microscope system according to any one of claims 2 to 17,
wherein the recommendation degree information includes at least one of at least one or more pieces of numerical information and one or more pieces of symbol information.

19. The microscope system according to any one of claims 1 to 18,
wherein the microscope is an inverted microscope including an eyepiece.

20. A projection unit used by being mounted on a microscope, comprising:
an image processing device that generates an auxiliary image including auxiliary information contributing to sperm sorting on the basis of a photographed image of a sample including sperm placed on a stage of the microscope; and
a superimposition device that superimposes the auxiliary image generated by the image processing device on an optical image of the sample formed on an image surface on an optical path of the microscope.

21. A sperm sorting support method comprising:
acquiring a photographed image of a sample including sperm placed on a stage of a microscope;
generating an auxiliary image including auxiliary information contributing to sperm sorting on the basis of the acquired photographed image; and
superimposing the generated auxiliary image on an optical image of the sample formed on an image surface on an optical path of the microscope.
